(19) **Europäisches Patentamt**
**European Patent Office**
**Office européen des brevets**

(11) **EP 3 367 814 B1**

(12) **EUROPEAN PATENT SPECIFICATION**

(45) Date of publication and mention
of the grant of the patent:
**21.08.2019 Bulletin 2019/34**

(21) Application number: **16791349.0**

(22) Date of filing: **27.10.2016**

(51) Int Cl.:
*A61K 36/87* (2006.01)    *A61K 36/45* (2006.01)
*A23K 40/30* (2016.01)    *A23K 10/30* (2016.01)
*A23K 20/111* (2016.01)    *A23K 50/42* (2016.01)
*A23K 40/25* (2016.01)    *A61P 39/06* (2006.01)
*A23K 50/40* (2016.01)    *A23K 10/00* (2016.01)

(86) International application number:
**PCT/EP2016/075920**

(87) International publication number:
**WO 2017/072225 (04.05.2017 Gazette 2017/18)**

(54) **SYNERGISTICALLY-EFFECTIVE ANTIOXIDANT COMPOSITION FOR PETS**

SYNERGISTISCH WIRKSAME ANTIOXIDANSZUSAMMENSETZUNG FÜR HAUSTIERE

COMPOSITION D'ANTIOXYDANTS À EFFICACITÉ SYNERGIQUE POUR ANIMAUX DE
COMPAGNIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.10.2015 US 201562246948 P
22.04.2016 EP 16305474**

(43) Date of publication of application:
**05.09.2018 Bulletin 2018/36**

(73) Proprietors:
• **Spécialités Pet Food
56250 Elven (FR)**
• **Activ'inside
33500 Libourne (FR)**

(72) Inventors:
• **LEPOUDERE, Anne
56380 Guer (FR)**
• **LETHUILLIER, Delphine
56400 Plougoumelen (FR)**
• **GAUDOUT, David
33360 Carignan de Bordeaux (FR)**
• **LEMAIRE, Benoît
33500 Libourne (FR)**
• **REY, Stéphane
26200 Montélimar (FR)**

(74) Representative: **Regimbeau
20, rue de Chazelles
75847 Paris Cedex 17 (FR)**

(56) References cited:
WO-A1-2007/141764    US-A1- 2008 213 401
US-A1- 2008 292 607    US-A1- 2010 021 533

• **With Douglas Laboratories: "Optimized
Curcumin with Neurophenol(TM)", , 1 January
2015 (2015-01-01), XP055270332, Retrieved from
the Internet:
URL:http://www.douglaslabs.com/media/DL202
028.pdf [retrieved on 2016-05-03]**
• **ANNE-SOPHIE MARTINEAU ET AL: "The
consumption of a grape and blueberry
polyphenol-rich mixture is safe in dogs", THE
FASEB JOURNAL; EXPERIMENTAL BIOLOGY
MEETING 2015; BOSTON, MA, USA; MARCH 28
-APRIL 01,2015, FEDERATION OF AMERICAN
SOCIETIES FOR EXPERIMENTAL BIOLOGY, US ,
vol. 29, no. 1, Suppl 1 April 2015 (2015-04-01),
XP002757332, ISSN: 0892-6638 Retrieved from
the Internet:
URL:http://www.fasebj.org/content/29/1_Sup
plement/136.1**
• **Super User: "Objectives", , 1 January 2013
(2013-01-01), XP055324594, Retrieved from the
Internet:
URL:http://www.neurophenols.org/index.php/
en/objectives [retrieved on 2016-11-30]**

**EP 3 367 814 B1**

**(Cont. next page)**

- JEREMY P. E. SPENCER: "The impact of fruit flavonoids on memory and cognition", BRITISH JOURNAL OF NUTRITION, vol. 104, no. S3, 1 October 2010 (2010-10-01), pages S40-S47, XP055243444, UK ISSN: 0007-1145, DOI: 10.1017/S0007114510003934
- WILHELMINA KALT ET AL: "Phenolics ofVaccinium berries and other fruit crops", JOURNAL OF THE SCIENCE OF FOOD AND AGRICULTURE, vol. 88, no. 1, 1 January 2008 (2008-01-01), pages 68-76, XP055126711, ISSN: 0022-5142, DOI: 10.1002/jsfa.2991
- G.K. JAYAPRAKASHA ET AL: "Antioxidant activity of grape seed (Vitis vinifera) extracts on peroxidation models in vitro", FOOD CHEMISTRY, vol. 73, no. 3, 1 May 2001 (2001-05-01), pages 285-290, XP055324750, NL ISSN: 0308-8146, DOI: 10.1016/S0308-8146(00)00298-3
- CHOU T C ET AL: "Quantitative analysis of dose-effect relationships: the combined effects of multiple drugs or enzyme inhibitors", ADVANCES IN ENZYME REGULATION, PERGAMON PRESS, OXFORD, GB, vol. 22, 1 January 1984 (1984-01-01), pages 27-55, XP023796270, ISSN: 0065-2571, DOI: 10.1016/0065-2571(84)90007-4 [retrieved on 1984-01-01]
- T.-C. CHOU: "Drug Combination Studies and Their Synergy Quantification Using the Chou-Talalay Method", CANCER RESEARCH, vol. 70, no. 2, 15 January 2010 (2010-01-15), pages 440-446, XP055169871, ISSN: 0008-5472, DOI: 10.1158/0008-5472.CAN-09-1947

Remarks:
The file contains technical information submitted after the application was filed and not included in this specification

**Description**

The present invention relates to the field of pet food.

More precisely, the invention concerns an antioxidant composition for use in pet food or pet supplement comprising an extract of *Vitis vinifera* and an extract of *Vaccinium angustifolium,* wherein said composition comprises resveratrol, at least 1 % of catechins and/or epicatechins and at least 5 ppm of ferulic acid, wherein the extract of *Vitis vinifera* is obtained from at least the skins of *Vitis vinifera,* wherein said extract of *Vitis vinifera* provides said resveratrol, catechins and/or epicatechins, and wherein said extract of *Vaccinium angustifolium* provides said ferulic acid.

## BACKGROUND OF THE INVENTION

Old age is well known to be associated with neurodegenerative disorders such as Alzheimer's disease (AD) and Parkinson's disease (PD).

Regarding pets, Cognitive Dysfunction Syndrome (CDS) is an Alzheimer's disease like disorder and is an age-related behavioral disorder which is observed in cats and dogs and is characterized by a decline in cognitive ability that cannot be attributed to an unrelated general medical condition such as neoplasia, infection, or organ failure. In dogs, symptoms of age-related behavioral disorders such as CDS include memory loss, which may be manifested by disorientation and/or confusion, altered interaction with family members, changes in sleep-wake cycle, decreased activity level and frequent inappropriate elimination. Similar symptoms can be observed in cats suffering from CDS.

The cause of CDS is unknown. Studies have shown that its symptoms increase with age, and many pathological changes occur in aging dogs and cats that can theoretically lead to CDS. Whatever the cause of CDS, it can dramatically affect the health and well-being of an animal suffering from it. Further, the companionship offered by a cat or dog with CDS can become less rewarding as the severity of the disease increases and its symptoms, such as depression, anxiety, and/or generally decreased health, become more severe.

Both healthy dogs and unhealthy dogs such as those diagnosed with CDS may present clinical troubles with progressive cognitive impairment and neuropathological changes (1) for example, memory disturbance, disorientation, loss of prior house-training, disturbances of sleep and wake cycles, altered social interactions and decreased activity.

In order to reverse, or at least minimize, age-related cognitive decline and to delay the onset of neurodegenerative diseases, nutrition-based preventive strategies are currently being contemplated. In particular, such strategies aim at avoiding or delaying the evolution towards dementia and thus maintaining a stable cognitive state and satisfactory wellness in elderly subjects. A growing body of evidence suggests that dietary interventions may delay or halt the progression of age-related health disorders and cognitive decline.

However, available dietary solutions are not especially adapted to pets. Only a few solutions are currently proposed as an efficient treatment of neurodegenerative disorders or as a solution to enhance cognitive functions and/or executive functions in pets.

Grapes are a rich source of monomeric phenolic compounds, such as catechins, epicatechins, procyanidins, and polymeric phenolic compounds such as oligoprocyanidins (from 2 to 10 units of monomers of flavanols) and tannins (>10 units of monomers of flavanols). Phenolic compounds in grapes and red wines have been reported to inhibit oxidation *in vitro.* Grapes and extracts from grapes are thus a widely efficient used antioxidant compound (2,3). Dietary supplements containing grapes molecules are currently proposed to humans.

However, acute renal failure has been reported in dogs after fresh grape consumption. Kidney histopathology has shown tubular and glomerular damages (4). Yet, the responsible factors of this condition are still unknown. In a retrospective study (4), dogs who ate different types of grapes presented clinical signs within 48h hours after consumption (vomiting, diarrhea, lethargy, oligo- or anuria) and biochemical abnormalities related to renal health compared to healthy dogs (higher plasma creatinine and blood urea nitrogen, an altered ionogram, glucose and proteins into urines).

As a consequence, the skilled person was absolutely not prompted to use grape in an antioxidant composition as a food or supplement, especially as a medicament, for dogs, especially for the treatment of neurodegenerative disorders or for enhancing their cognitive functions and/or executive functions.

A product named "Optimized Curcumin with Neurophenol™" and commercialized by Douglas Laboratories, contains LONGVIDA® Optimized Curcumin Extract, and Neurophenol™ blend providing 85 mg of flavonoids from grape extract (Vitis Vinifera) and blueberry extract (Vaccinium Angustifolium).

US2010/021533 relates to a synergistic mixture capable of improving a person's well being, lowering the risks of cardiovascular and/or Alzheimer's diseases and/or lowering blood sugar using natural and synthetic ingredients, such as, in a particular embodiment, grape (*Vitis Labrusca* or *Vitis rotundifolia*) and blueberry.

WO2007/141764 discloses a composition for promoting eye health in a companion animal and which comprises at least one polyphenol such as blueberry extract or grapeseed extract.

Moreover, numerous commercially-available antioxidant compositions are not palatable to pets. It is also well known that bitterness and astringency are sensorial characteristics associated with phenolic compounds. Pets, such as dogs and cats, are very sensitive to bitter taste which is perceived at very low concentration. This can lead to difficulties upon orally administering antioxidant compositions to pets.

An object of the present invention is to provide new antioxidant compositions that can be useful as a medicament, especially for the treatment of neurodegenerative disorders.

Another object of the present invention is to provide new antioxidant compositions that can be useful for enhancing cognitive functions and/or executive functions.

Another object is to provide uses of new antioxidant compositions having an enhanced efficiency and/or an enhanced capacity to improve memory of pets.

Another object is to provide new antioxidant compositions comprising an extract of grape and which are tolerated by pets, especially dogs.

Another object is to provide new antioxidant compositions which do not decrease palatability to pets when added to pet foods or pet supplements.

Another object of the present invention is to provide pet foods or pet supplements that can comprise these antioxidant compositions which can be active when orally administered to pets.

## SUMMARY OF THE INVENTION

The present invention concerns an antioxidant composition for use in pet food or pet supplement comprising an extract of *Vitis vinifera* and an extract of *Vaccinium angustifolium,* wherein said antioxidant composition comprises resveratrol, at least 1 % of catechins and/or epicatechins and at least 5 ppm of ferulic acid, wherein the extract of *Vitis vinifera* is obtained from at least the skins of *Vitis vinifera,* wherein said extract of *Vitis vinifera* provides said resveratrol, catechins and/or epicatechins, and wherein said extract of *Vaccinium angustifolium* provides said ferulic acid.

The present disclosure describes this synergistically-effective antioxidant composition for use as a medicament for pets, in particular in a method for treating a neurodegenerative disorder in a pet.

The present disclosure also describes the use of the synergistically-effective antioxidant composition for enhancing cognitive functions and/or executive functions in a pet, in particular for enhancing memory, attention, concentration, alertness, learning, intelligence, language, mood, stress, anxiety, vision and /or sleep.

The present disclosure also describes a method of preparing such pet food or pet supplement and a method of preparing such a synergistically-effective antioxidant composition.

## BRIEF DESCRIPTION OF THE FIGURES

The present invention is illustrated by reference to the following accompanying figures:

**Figure 1**: $\Delta$ Total antioxidant status ($\Delta$TAS as defined below, mmol/L) after 28 days supplementation of Mix, grape extract (Gra), blueberry extract (Blu), and maltodextrin (Control) in adult dogs. Bars within no common superscript (a, b and/or c) are statistically different, $p < 0.10$.

**Figure 2**: Number of dogs which provided better or worse sum of scores (20' + 90') compared to a baseline.

**Figure 3:** Kinetics of mix absorption comparing pet supplement vs dry pet food inclusion administration. Plasma polyphenol biomarkers (Mean $\pm$ SEM) from 6 adult dogs after a 7 days wash in period.

**Figure 4:** Plasma and urinary early and specific renal biomarkers (Mean $\pm$ SEM) taken from 4 groups of adult dogs (n = 6) feeding a experimental diet supplemented with different concentrations of a "mix", i.e. an antioxidant composition according to the invention (Control, Maltodextrin; Mix 1, 4 mg /kg BW/day; Mix 5, 20 mg /kg BW/day; Mix 10, 40 mg /kg BW/day) during 6 months.

## DEFINITIONS

Unless specifically stated otherwise, percentages are expressed herein by weight of a product reference (in particular, an antioxidant composition or a pet food or a pet supplement).

In the present disclosure, ranges are stated in shorthand, so as to avoid having to set out at length and describe each and every value within the range. Any appropriate value within the range can be selected, where appropriate, as the upper value, lower value, or the terminus of the range. For example, a range of 0.1-1.0 represents the terminal values of 0.1 and 1.0, as well as the intermediate values of 0.2, 0.3, 0.4, 0.5, 0.6, 0.7, 0.8, 0.9, and all intermediate ranges encompassed within 0.1-1.0, such as 0.2-0.5, 0.2-0.8, 0.7-1.0, etc. Moreover, the term "at least" encompasses the hereafter cited value. For example, "at least 5 %" has to be understood as also encompassing "5%".

Moreover, in the present invention, measurable values, such as an amount, have to be understood as encompassing standard deviations which can easily be determined by the skilled person in the technical domain of reference. Preferably, these values are meant to encompass variations of ±5%.

As used throughout, the singular form of a word includes the plural, and vice versa, unless the context clearly dictates otherwise. Thus, the references "a", "an", and "the" are generally inclusive of the plurals of the respective terms. For example, reference to "a method" or "a food" includes a plurality of such "methods" or "foods". Similarly, the words "comprise", "comprises", and "comprising" are to be interpreted inclusively. Likewise the terms "include", "including" and "or" should all be construed to be inclusive. All these terms however have to be considered as encompassing exclusive embodiments that may also be referred to using words such as "consist of'.

The term "ppm" is herein used according to its conventional meaning. More precisely, it refers herein to a weight amount relative to the total weight of the antioxidant composition (mg/kg) (unless otherwise indicated).

The methods and compositions and other embodiments exemplified here are not limited to the particular methodologies, protocols, and reagents that are described herein because, as the skilled artisan will appreciate, they may vary.

Unless defined otherwise, all technical and scientific terms, terms of art, and acronyms used herein have the meanings commonly understood by the skilled artisan in the field(s) of the invention, or in the field(s) where the term is used. Although any compositions, methods, articles of manufacture, or other means or materials similar or equivalent to those described herein can be used in the practice of the present invention, the preferred compositions, methods, articles of manufacture, or other means or materials are described herein.

The terms "pet", "companion animal" and "animal" are synonymous and mean any domesticated animal including, without limitation, cats, dogs, rabbits, guinea pigs, ferrets, hamsters, mice, gerbils, birds, horses, cows, goats, sheep, donkeys, pigs, and the like, and preferably cats or dogs. In the context of the present invention, dogs are preferred.

In a particular embodiment of the invention, the pet is "aging" or "aged", which preferably means that said pet has reached or exceeded 50% of the average life expectancy for the pet's species and/or breed within such species. For example, if the average life expectancy for a given breed of dog is 12 years, then an "aging animal" within that breed is 6 years old or older.

The terms "food", "diet", "pet food", "pet food product", and "food product" as used herein are synonymous. These terms mean a product or composition that is eaten by an animal and provides at least one nutrient to the animal. More specifically, a "food" is here a "nutritionally-balanced food".

Nutritionally-balanced pet foods are widely known and used in the art. A "nutritionally-complete", "nutritionally-balanced" or "complete and nutritionally-balanced food" is one that contains all known required nutrients for the intended recipient or consumer of the food, in appropriate amounts and proportions based, for example, on recommendations of recognized or competent authorities in the field of companion animal nutrition. Such foods are therefore capable of serving as a sole source of dietary intake to meet essential needs of pets, without the addition of supplemental nutritional sources.

The term "pet food" refers to any food that may be in any form, solid, dry, wet, semi-moist or combinations thereof There are three main categories or classes of pet foods depending on their moisture content, which is either low or medium or high:

- dry or low moisture-containing products (having less than about 14% moisture), such as kibbles: they usually produce a crunching sound when chewed by a pet; they are generally highly nutritious, may be packaged, e.g., in bags or boxes, and are highly convenient to store and use;

- canned or wet or high moisture-containing products (having more than about 50% moisture), such as chunk-in-"X" products : typically high meat-containing products ; they are usually costly to produce and package (mainly in cans);

- semi-moist or semi-dry or soft dry or soft moist or intermediate or medium moisture-containing products (having from about 14% to about 50% moisture), such as loafs: usually packaged in appropriate bags or boxes.

The term "kibble" used herein refers to particulate chunks or pieces formed by either a pelleting or extrusion process. Typically, kibbles are produced to give dry and semi-moist pet food, preferably dry pet food. The pieces can vary in sizes and shapes, depending on the process or the equipment. For instance, kibbles can have spherical, cylindrical, oval, or similar shapes. They can have a largest dimension of less than about 2 cm for example.

The term "chunk-in-"X" products" mean herein all edible foodstuffs comprising chunks in a preparation (said preparation being "the X preparation"). Classical examples thereof are chunk-in-jelly products, chunk-in-gravy products, and the like. This category of "chunk-in-X" products encompasses also edible forms other than chunks that may be contained in the X preparation such as a jelly, a gravy, and the like. For instance, other forms than chunks may be sliced products, grated products, etc.

The term "loaf' used herein refers to edible foodstuffs obtained as moist products, and includes terrines, pâtés, mousses, and the like.

The term "treat" (or "biscuit") means any food item that is designed to be fed to a pet, preferably at non-meal time, by the owner to help, promote or sustain a bonding process between a pet and its owner. Examples of treats for dogs are bones. Examples of treats for cats are stuffed pillows and chewable sticks.

The term "pet supplement" or "supplement" or "food supplement" or "dietary supplement" means a product that is intended to be ingested in addition to the normal animal diet. Supplements may be in any form, e.g., solid, liquid, gel, tablets, capsules, powder, and the like. Preferably, they are provided in convenient dosage forms. In some embodiments, they are provided in bulk consumer packages such as bulk powders, liquids, gels, or oils. In other embodiments, supplements are provided in bulk quantities to be included in other food items such as snacks, treats, supplement bars, and the like. Palatability-enhancing compositions can be used to improve palatability of dietary supplements in the same manner as they are used to improve palatability of nutritionally-balanced foods.

The term "antioxidant composition" refers to a composition having in particular an inhibitory effect on biological oxidative processes involving free radicals or singlet oxygen and/or on specific gene expression pathways involved in the regulation of the oxidative state and inflammatory status. More particularly, an antioxidant composition according to the invention significantly decreases the effects of reactive species, such as reactive oxygen and nitrogen species, on physiological function in pets.

The term "synergistically-effective" or "synergistic" means that at least some of the compounds of an antioxidant composition of the invention function synergistically to inhibit biological oxidation involving free radicals or singlet oxygen. More specifically, an extract of *Vitis vinifera* and an extract of *Vaccinium angustifolium* as herein defined demonstrate significantly better antioxidant effects when used in combination than when used alone.

Hereinafter and for sake of clarity, a "synergistically-effective antioxidant composition" may be shortly referred to as an "antioxidant composition".

The term "anthocyanin" refers to a class of flavonoids, which may be glycosylated (such as cyanidine, malvidine, delphinidine, pelargonidine, peonidine, petunidine) or not glycosylated.

The term "proanthocyanidin" are polymers which are classified into the class of flavones, and more particularly flavanols. They encompass oligomers (oligoprocyanidins or OPC) having a degree of polymerization of 2 to 10, and polymers having a degree of polymerization greater than 10.

The term "extract of *Vitis Vinifera"* refers to at least one molecule, preferably a mix of molecules, obtained from grape *Vitis Vinifera,* issued of the species of *Vitis.* In the present invention, the raw material of *Vitis Vinifera* is preferably obtained from the fruits and/or leafs and/or seeds and/or woods, and more preferably skins and seeds.

In a particular embodiment, an extract of *Vitis Vinifera* does not contain oligomers and polymers having a degree of polymerization superior to 12, preferably superior to 10. Thus, an extract of *Vitis vinifera* preferably consists essentially of monomers and oligomers having a degree of polymerization between 2 and 12, preferably between 2 and 10. By "consists essentially of', it is meant that the liquid extract of *Vitis vinifera* contains less than 0.5%, preferably less than 0.1% of oligomers and polymers having a degree of polymerization superior to 12, preferably superior to 10 (% based on total phenolic compounds extract). Typically, monomers of an extract of *Vitis vinifera* include at least catechins and/or epicatechins, preferably catechins and epicatechins. Polymers which are preferably not present in an extract of *Vitis Vinifera* comprise tannins.

The term "extract of *Vaccinium angustifolium"* refers to at least one molecule, preferably a mix of molecules, obtained from blueberry *Vaccinium angustifolium.* In the present invention, the raw material of *Vaccinium angustifolium* is preferably obtained from leafs and/or fruits, more preferably from leafs and fruits. Typically, an extract of *Vaccinium angustifolium* can be obtained in the residual materials arising from juice pressing activities of blueberries.

When referring to "an extract of *Vitis vinifera* and an extract of *Vaccinium angustifolium"* or to "a combination of an extract of *Vitis vinifera* and an extract of *Vaccinium angustifolium",* it refers either to a mix of one or more extracts of *Vitis vinifera* and one or more extracts of *Vaccinium angustifolium* which have been obtained separately and mixed together, or it refers to one or more extracts obtained from both *Vitis vinifera* and *Vaccinium angustifolium.* In other terms, the process of extraction can be performed separately on extract(s) of *Vitis vinifera* and on extract(s) of *Vaccinium angustifolium,* or it can be performed on a mix of extract(s) of both *Vitis vinifera* and *Vaccinium angustifolium,* or it can be a combination of these embodiments. These extracts may be mixed with an appropriate carrier, for example at least a maltodextrin.

When referring to «catechins and/or epicatechins », it refers to monomers of flavanols. In particular, it refers to either catechins alone, or epicatechins alone or a mix of catechins and epicatechins, preferably it refers to a mix of catechins and epicatechins.

When referring to « quercetin », it refers either to quercetin alone, or quercetin glycosides alone or a mix of quercetin and quercetin glycosides, preferably it refers to a mix of quercetin and quercetin glycosides. Quercetin glycosides can include for example quercetin glucoside, quercetin galactoside, quercetin rhamnoside, quercetin xyloside, quercetin arabinoside, rutin, and the like.

The term "treatment" and derived terms mean reversing, alleviating, stopping or preventing a neurodegenerative disorder as defined in the context of the invention and/or at least one symptom linked to said disorder. The term "treatment" also refers to a prophylactic treatment which can delay the onset of the above-mentioned diseases or disorders. Typically, a "method of treating a neurodegenerative disorder" embraces prevention or treatment of a neurodegenerative disorder.

The term "Alzheimer's disease like disorders in pets" herein refers to a disease in similar to Alzheimer's disease in humans. For example, aged dogs develop neuropathology that is related to that seen in both successfully aging humans and patients with Alzheimer's disease, such as beta amyloid protein (5). However, dogs do not demonstrate every hallmark of Alzheimer's disease, in particular, tau-containing neurofibrillar tangles have not been observed. Therefore, the condition in dogs is preferably referred to as Cognitive Dysfunction Syndrome (CDS). In a particular embodiment of the present invention, the neurodegenerative disorder is thus an Alzheimer's disease like disorders in pets, more preferably Cognitive Dysfunction Syndrome.

A "therapeutically effective amount" of an antioxidant composition or of a pet food or pet supplement, as used herein, is a quantity of the antioxidant composition or pet food or pet supplement provided by a particular route of administration and at a particular dosing regimen, that is sufficient to achieve a desired therapeutic and/or prophylactic effect as above defined. The amount of the composition/pet food/pet supplement administered to the pet will depend on the type and severity of the disease, the activity of the specific compound employed; the specific composition/pet food/pet supplement employed, the type, age, body weight, general health, sex and diet of the pet; the time of administration, route of administration, and rate of excretion of the specific compound employed; the duration of the treatment; drugs used in combination or coincidential with the composition/pet food/pet supplement; and like factors well known in the medical arts. The skilled artisan will be able to determine appropriate dosages depending on these and other factors. For example, it is well known within the skill of the art to start doses of the compound at levels lower than those required to achieve the desired therapeutic effect and to gradually increase the dosage until the desired effect is achieved.

The term "medicament" herein refers to a veterinary product that can be administered to pets in very different ways depending on their mode of action and their ability to be taken up by the treated pet. Thus a medicament can be administered, for example, topically as pour-on or spot-on formulations, in form of shampoos, showers, as a dip, bath or spray, in form of a collar, and in many variants of these application forms. They can also be administered systemically, for example, orally, parenterally and in certain cases even transdermally. Examples of oral administration are in a pet food or in a pet supplement as defined above. Each of these administration forms can have advantages or disadvantages depending on the actual situation and the pet that is in need of such a treatment.

As used herein, the term "palatability" or "palatability effect" refers to the overall willingness of a pet to eat a certain pet food or pet supplement. Whenever a pet shows a preference, for example, for one of two or more pet foods, the preferred pet food is more "palatable", and has "enhanced palatability". Such preference can arise from any of the pet's senses, but typically is related to, *inter alia,* taste, aroma, flavour, texture, smell and/or mouth feel.

Different methods exist to determine a palatability effect. Examples of such methods involve exposure of pets to pet foods or pet supplements either simultaneously (for example, in side-by-side, free-choice comparisons, e.g., by measuring relative consumption of at least two different pet foods), or sequentially (e.g., using single bowl testing methodologies). At least two different methods may be used to consolidate the thus obtained results on the palatability effect of a given pet food or pet supplement. "Initial appeal" or "attractiveness" is an aspect of palatability that induces an animal to initially taste or try a food or supplement, and that can be measured by the criteria "first choice" or "first food consumed". This criterion identifies which food first attracts the pet to eat and is expressed as an absolute value. "Continued consumption" is an aspect of palatability that induces an animal to continue consuming a food that has been initially only tasted or tried. Continued consumption can be evaluated by determining a "consumption ratio" or "intake ratio". The "consumption ratio" measures the consumption of one food relative to another and is expressed as a relative value (see a detailed description of a method for assessing palatability below).

As used herein, a "pet food ingredient" is any compound, composition or material that is suitable for pet consumption. Non-limiting examples of pet food ingredients are proteins, peptides, amino acids, grains, carbohydrates, fats or lipids, nutrients, palatability enhancers, animal digests, meat meals, gluten, preservatives, surfactants, texturing or texturizing or stabilizing agents, colouring agents, inorganic phosphate compounds, flavors, seasonings, etc.

As used herein, a "pet food preparation" is any compound, composition or material that is used for preparing food products for pet consumption, i.e. pet food products. Typically, a pet food preparation includes at least one pet food ingredient. Such ingredients may be comprised as such in the pet food preparation, or they can be contacted into the preparation and react *in situ* for producing transformed materials that are also encompassed in the group of ingredients of a "pet food preparation". Examples of ingredients that react together in the preparation are, without limitation, nitrogen compounds and carbohydrates, so as to obtain transformed materials such as Maillard reaction products, and the like.

The term "animal digest" means herein material which results from chemical and/or enzymatic hydrolysis of clean, undecomposed animal tissue. In some embodiments, an animal digest as used herein is fully consistent with the definition promulgated by the Association Of American Feed Control Officials, Inc. (AAFCO). Animal digest is preferably derived from animal tissues, including cold-blooded marine animals, excluding hair, horns, teeth, hooves, and feathers. The skilled artisan will appreciate that while such tissues are not preferred, trace amounts might be found unavoidably even under good manufacturing practices. Also not included are visceral contents or foreign or fecal matter, although trace contaminant amounts are sometimes present. An animal digest may be dried or not. Examples of animal digests are:

- digest of poultry (or pork, beef, sheep, lamb, fish, etc): material from poultry (pork, beef, etc) which results from chemical and/or enzymatic hydrolysis of clean and undecomposed tissue;

- digest of pork (or beef, sheep, lamb, fish, etc) by-products: material from pork (beef, etc.) which results from chemical and/or enzymatic hydrolysis of clean and undecomposed tissue from non-rendered clean parts from cattle (pigs, sheep, lamb, etc), other than meat, for example lungs, spleen, kidneys, brain, livers, blood, bone, partially-defatted low-temperature fatty tissue, and stomachs and intestines, freed of their contents;

- digest of poultry by-products: material which results from chemical and/or enzymatic hydrolysis of clean and undecomposed tissue from non-rendered clean parts of poultry, other than meat, such as livers, hearts, heads, feet, and viscera. As used herein, "poultry" encompasses any species or kind of bird, preferably chicken, turkey, duck, and the like; and

- digest of fish by-products: material which results from chemical and/or enzymatic hydrolysis of clean and undecomposed tissue from non-rendered clean parts from fish. As used herein, "fish" encompasses any species or kind of fish or crustaceans, preferably tuna, salmon, cod, whitefish, shrimp, sardine, and the like.

Animal digests may also be referred to as "animal products" or "animal by-products", all these terms being used herein as synonymous.

"Proteins" include all conventional protein sources that are compatible for animal consumption, especially plant or vegetable proteins, animal proteins (such as casein or albumin or animal digests or meat meals), and microbial proteins (such as yeast or fungi or algal). The term "meat meal" means the meal obtained from carcasses of the above mentioned animals, i.e. poultry, pork, beef, sheep, lamb, fish.

"Protein" also includes protein hydrolysates, the degree of hydrolysis of which can be controlled depending on the objective.

The term "yeast" herein refers to any yeast, preferably inactive, as well as to yeast by-products that are compatible with compositions for animal consumption. Yeasts are well known in the art as being protein-rich. Yeasts include, without limitation, brewer's yeast, baker's yeast, torula yeast, molasses yeast, and the like. Yeast by-products include, without limitation, yeast extracts, yeast hydrolysates, cream yeasts, etc.

Examples of fats include tallow, oils, from any origin such as animal, plant (including vegetable), or marine oils. Plant oils which are available in large quantities are typically canola oil, soybean oil, corn oil, olive oil, sunflower oil, linseed oil, palm oil, safflower oil, and the like, as well as by-products thereof. Typical animal fats are tallow, lard, poultry fat, and the like, as well as by-products thereof. Marine oils are typically tuna oil, sardine oil, salmon oil, anchovy oil, fish oil, and the like, as well as by-products thereof. Also are encompassed herein the fats that are derived from animal, plant, marine sources, or that are produced by animals and plants.

The term "grain" means a cereal grain that may be used as a source of nutrients (e.g., proteins, starch, minerals, and vitamins). Examples of grains are corn, milo, alfalfa, wheat, barley, rice, soy, and the like.

The term "gluten" means a protein fraction from wheat, corn, rye, barley, oats or their crossbred varieties and derivatives thereof.

The terms "palatability enhancers", "palatants", "flavours", "palatability agents", "appetizing factors", "flavour compositions", "palatability-enhancing compositions", "flavour enhancers", and any other similar terms mean any compound, composition or material that is suitable for pet consumption material and that enhances the palatability of a pet food or a pet supplement to an animal.

A palatability enhancer may be a single material or a blend of materials, and it may be natural, processed or unprocessed, synthetic, or part of natural and part of synthetic materials. Non-limiting examples of palatability-enhancing ingredients of palatability enhancers are animal digests, vegetarian palatability-enhancing composition ingredients, Maillard ingredients, Maillard reaction products, proteins, peptides, amino acids, carbohydrates, fats, nutrients, preservatives, surfactants, texturing agents, flavors, etc. Ingredients may be comprised in a palatability-enhancer, or they can be contacted into the pet food preparation and react *in situ* for producing transformed materials that are also encompassed by the term "palatability-enhancer". Examples of ingredients that react together in the composition are, without limitation, fats, peptides, amino acids, and carbohydrates, so as to obtain transformed materials such as Maillard reaction products, and the like.

As used herein, the term "Maillard reaction product" means herein any compound produced by a Maillard reaction. In particular, a Maillard reaction product is a compound that provides flavour and/or color and/or odor and/or taste and/or aftertaste.

The term « vegetarian palatability-enhancing composition ingredients » means herein materials free of meat or animal products, and derived from or isolated from plant, bacterial, fungal or algal sources, or single compounds not obtained from animal sources. Vegetarian palatability-enhancing composition ingredients can be dry or liquid.

Examples of carbohydrates include dextrose, fructose, sucrose, polysaccharides, fibers, starches, and the like.

"Fiber source" or "dietary fiber" refers to food ingredients corresponding to components of a plant that are resistant to digestion by animal's digestive enzymes. Fiber can be soluble or insoluble. Sources of dietary fiber for use in the diets disclosed herein include, but are not limited to, beet pulp, guar gum, chicory root, psyllium, cellulose, wheat oat, corn bran, flax seed, and the like.

Examples of nutrients include, without limitation, vitamins, minerals and electrolytes, such as vitamins A, C, E, B12, D3, folic acid, D-biotin, cyanocobalamin, niacinamide, thiamine, riboflavin, pyridoxine, menadione, beta-carotene, calciumpantothenate, choline, inositol, calcium, potassium, sodium, zinc, iron, manganese, copper, iodine, and the like.

Surfactants, seasonings, texturing agents or texturizing agents, preservatives, stabilizing agents which may be used in the context of the present invention are well known by the skilled person.

"Coating", as used herein, refers to the topical deposition of a composition, such as a antioxidant composition of the invention and/or one or more palatability-enhancer and/or fat, onto the surface of the pet food or the pet supplement, such as by spraying, dusting, and the like. The antioxidant composition of the invention may be added to a pet food or a pet supplement by coating, typically in a mixture with one or more palatability-enhancer and/or fat.

"Inclusion" as used herein, refers to the addition of a composition, such as an antioxidant composition of the invention, in the core of a pet food or pet supplement. For example, inclusion of a composition in a pet food can be made by mixing it with other pet food ingredients, before further processing steps for obtaining the final pet food product (including thermal treatment and/or extrusion and/or retorting, etc).

"Containers" include, but are not limited to, bags, boxes, cartons, bottles, packages of any type or design or material, over-wrap, shrink-wrap, stapled or otherwise affixed components, or combinations thereof, that are used to store materials.

The term "single package" means that the components of a kit are physically associated in or with one or more containers and considered a unit for manufacture, distribution, sale, or use. A single package may be containers of individual components physically associated such that they are considered a unit for manufacture, distribution, sale, or use.

As used herein, a "means for communicating information or instructions" is a kit component under any form suitable for providing information, instructions, recommendations, and/or warranties, etc. Such a means can comprise a document, digital storage media, optical storage media, audio presentation, visual display containing information. The means of communication can be a displayed web site, brochure, product label, package insert, advertisement, visual display, etc.

## DESCRIPTION OF SOME EMBODIMENTS OF THE INVENTION

The present invention is defined in appended claims 1 to 14.

The inventors showed for the first time that a combination of an extract of *Vitis vinifera* and an extract of *Vaccinium angustifolium,* comprising resveratrol, at least 1 % of catechins and/or epicatechins and at least 5 ppm of ferulic acid, wherein the extract of *Vitis vinifera* is obtained from at least the skins of *Vitis vinifera,* wherein said extract of *Vitis vinifera* provides said resveratrol, catechins and/or epicatechins, and wherein said extract of *Vaccinium angustifolium* provides said ferulic acid, efficiently and synergistically protects pets against oxidative stress.

Moreover, it has been demonstrated that such an antioxidant composition combining an extract of *Vitis vinifera* and an extract of *Vaccinium angustifolium* is well tolerated by pets, especially by dogs, contrary to what the skilled person would have anticipated regarding the published studies on effect of grapes on dogs.

Furthermore, it has surprisingly been demonstrated that a pet food or a pet supplement comprising such an antioxidant composition according to the invention does not decrease the palatability to pets compared to a pet food or a pet supplement which does not comprise such a composition.

Finally, the inventors have observed that the combination of an extract of *Vitis vinifera* and an extract of *Vaccinium angustifolium* comprising the specific amounts of molecules as claimed enhances the bioavailability of these molecules, in particular polyphenols, compared to the bioavailability obtained when added alone. Advantageously, said bioavailability is synergistically enhanced.

The above compositions are particularly useful wherein the oxidative status of the companion animal can benefit from the antioxidant composition, such as young animals in the growth stage, mature animals in need of antioxidants for preventing development of disease states related to oxidative stress, and in aging animals already demonstrating health issues due to oxidative stress, such as decreased cognitive abilities.

Moreover, the inventors have demonstrated that this combination of an extract of *Vitis vinifera* and an extract of *Vaccinium angustifolium* significantly improves memory performances of pets, especially working memory. Thus, the antioxidant composition is an efficient treatment for neurodegenerative disorders of pets or for enhancing cognitive functions and/or executive functions in a pet.

The present disclosure concerns a pet food or pet supplement comprising a synergistically-effective antioxidant composition comprising an extract of *Vitis vinifera* and an extract of *Vaccinium angustifolium,* wherein said antioxidant composition comprises at least 1 % of catechins and/or epicatechins and at least 5 ppm of ferulic acid.

As previously mentioned, percentages are expressed herein by weight of the antioxidant composition.

Typically, the pet food is a kibble, a treat, a loaf or a chunk-in-X product, more preferably a kibble, and even more preferably a dry kibble.

In a particular example in which the pet food is a kibble, an antioxidant composition of the invention may be comprised in the core of the kibble and/or in a coating of the kibble.

The present invention concerns a (synergistically-effective) antioxidant composition for use in pet food or pet supplement comprising an extract of *Vitis vinifera* and an extract of *Vaccinium angustifolium,* wherein said composition comprises resveratrol, at least 1 % of catechins and/or epicatechins and at least 5 ppm of ferulic acid, wherein the extract of *Vitis vinifera* is obtained from at least the skins of *Vitis vinifera,* wherein said extract of *Vitis vinifera* provides said resveratrol, catechins and/or epicatechins, and wherein said extract of *Vaccinium angustifolium* provides said ferulic acid.

The antioxidant activity or the antioxidant capacity can be determined by measuring total antioxidant status. Analytical methods known by the skilled person can be used to quantitatively measure the total antioxidant status (TAS). For example, a colorimetric-based assay using ABTS could be used, such as the one used in the Examples.

An antioxidant composition for use according to the invention comprises at least 1% of catechins and/or epicatechins, preferably at least 2% of catechins and/or epicatechins, more preferably at least 3% of catechins and/or epicatechins, more preferably at least 4% of catechins and/or epicatechins, more preferably at least 5% of catechins and/or epicatechins, more preferably at least 5.5% of catechins and/or epicatechins, more preferably at least 5.6% of catechins and/or epicatechins.

In a particular embodiment, the antioxidant composition for use according to the invention comprises less than 30%, more preferably less than 25%, more preferably less than 20% of catechins and/or epicatechins.

An antioxidant composition for use according to the invention comprises at least 5 ppm of ferulic acid, preferably at least 10 ppm of ferulic acid, more preferably at least 15 ppm of ferulic acid, more preferably at least 20 ppm of ferulic acid.

In a particular embodiment, the antioxidant composition for use according to the invention comprises less than 500 ppm of ferulic acid, more preferably less than 400 ppm of ferulic acid, more preferably less than 300 ppm of ferulic acid, more preferably less than 200 ppm of ferulic acid, more preferably less than 100 ppm of ferulic acid, more preferably less than 50 ppm of ferulic acid.

An antioxidant composition according to the invention comprises at least a mix of molecules, typically obtained from an extract of *Vitis vinifera* and an extract of *Vaccinium angustifolium,* and comprising at least resveratrol, catechins and/or epicatechins, and ferulic acid. This antioxidant composition preferably comprises at least resveratrol, catechins and/or epicatechins, and ferulic acid, and at least one molecule selected from the group consisting of:

- anthocyanins, and/or
- quercetin.

The extract of *Vitis vinifera* provides at least catechins and/or epicatechins, and further provides resveratrol.

The extract of *Vitis vinifera* is obtained from at least the skins of *Vitis vinifera.* Indeed, skins of *Vitis vinifera* are rich in resveratrol, contrary to seeds of *Vitis vinifera* which only comprise traces of resveratrol (negligible amount, or insignificant amount, or undetectable amount). Preferably, the extract of *Vitis vinifera* is obtained from the skins and the seeds of *Vitis vinifera.* Indeed, the seeds are rich in monomers of flavanols (in particular epicatechins and catechins) and oligoprocyanidins.

Advantageously, said extract of *Vitis vinifera* comprises less than 0.5%, more preferably less than 0.1%, of polymers of flavanols, in particular proanthocyanidins, having a degree of polymerization greater than 10. This provides the advantage of enhancing bioavailability of the molecules of the composition, such as anthocyanins.

The extract of *Vaccinium angustifolium* provides at least ferulic acid, and preferably further provides anthocyanins and/or quercetin, preferably anthocyanins and quercetin.

In a preferred embodiment, an antioxidant composition according to the invention comprises at least 4000 ppm of resveratrol, preferably at least 4500 ppm of resveratrol, more preferably at least 5000 ppm of resveratrol, more preferably at least 5100 ppm of resveratrol, more preferably at least 5200 ppm of resveratrol, more preferably at least 5300 ppm of resveratrol.

In a particular embodiment, the antioxidant composition for use according to the invention comprises less than 8000 ppm of resveratrol, more preferably less than 7000 ppm of resveratrol.

More preferably, the ratio A/B, wherein A is resveratrol and B is catechins and/or epicatechins (both being expressed in % of the antioxidant composition), is of at least 0.005, preferably at least 0.01, more preferably at least 0.02, more preferably at least 0.03, more preferably at least 0.04, more preferably at least 0.05, more preferably at least 0.06, more preferably at least 0.07, more preferably at least 0.08, more preferably at least 0.09.

In a preferred embodiment, an antioxidant composition according to the invention comprises at least 500 ppm of anthocyanins, preferably at least 600 ppm of anthocyanins, more preferably at least 650 ppm of anthocyanins, more preferably at least 700 ppm of anthocyanins.

In a particular embodiment, the antioxidant composition for use according to the invention comprises less than 2000 ppm of anthocyanins, more preferably less than 1000 ppm of anthocyanins, more preferably less than 900 ppm of anthocyanins, more preferably less than 800 ppm of anthocyanins.

In another particular embodiment, the antioxidant composition for use according to the invention comprises at least 200 ppm of malvidine-3-glucoside. Preferably, the anthocyanins comprise at least 25% of malvidine-3-glucoside.

In another preferred embodiment, an antioxidant composition according to the invention comprises at least 50 ppm of quercetin, preferably 60 ppm of quercetin, more preferably 65 ppm of quercetin, more preferably 70 ppm of quercetin, more preferably 75 ppm of quercetin, more preferably 77 ppm of quercetin.

In a particular embodiment, the antioxidant composition for use according to the invention comprises less than 1000 ppm of quercetin, more preferably less than 500 ppm of quercetin, more preferably less than 300 ppm of quercetin, more preferably less than 200 ppm of quercetin, more preferably less than 200 ppm of quercetin.

Determination of the amount of each of the different molecules may be determined by LC-DAD (Liquid chromatography-diode array detection), LC-fluorescence (Liquid chromatography-fluorescence detection) or LC-MS (Liquid chromatography- Mass spectrometry) or UPLC-MS/MS (Ultra performance liquid chromatography tandem Mass spectrometry), preferably by UPLC-MS/MS. Other measurements that the skilled person usually uses are available as well.

A synergistically-effective antioxidant composition can be prepared using any method known by the skilled person and which is appropriate for the present invention.

The present disclosure further relates to a method for preparing a synergistically-effective antioxidant composition comprising the steps consisting of:

a) preparing an extract of *Vitis vinifera;*
b) preparing an extract of *Vaccinium angustifolium;*
c) combining said extract of *Vitis vinifera* and *Vaccinium angustifolium* in order to obtain a synergistically-effective antioxidant composition comprising at least resveratrol, catechins and/or epicatechins, and ferulic acid, and preferably at least one molecule selected from the group consisting of anthocyanins, and/or quercetin.

An extract of *Vitis vinifera* provides at least catechins and/or epicatechins, and further provides resveratrol.

In a particular embodiment, the extract of *Vitis vinifera* has an amount of polymers of flavanols, in particular proanthocyanidins, having a degree of polymerization greater than 10 which is inferior to 0.5% by weight of total polyphenols.

Advantageously, the polymers of flavanols, in particular proanthocyanidins, of said extract of *Vitis vinifera* consist essentially of monomers and oligomers having a degree of polymerization between 2 and 12, preferably between 2 and 10.

An extract of *Vaccinium angustifolium* provides at least ferulic acid, and typically further provides anthocyanins and/or quercetin, preferably anthocyanins and quercetin.

In a particular embodiment, step a) comprises the steps consisting of:

a1) providing a raw material of *Vitis vinifera,* preferably consisting of skins and seeds of *Vitis vinifera,*
a2) adding said raw material of *Vitis vinifera* in a solvent, preferably an aqueous solution and/or ethanol, in order to provide a liquid slurry of *Vitis vinifera,*
a3) separating the liquid fraction from said slurry of *Vitis vinifera,* in order to provide a liquid solvent-containing extract of *Vitis vinifera,*
a4) eliminating the solvent from said liquid solvent-containing extract of *Vitis vinifera,* in order to provide a liquid extract of *Vitis vinifera,*
a5) optionally purifying said liquid extract of *Vitis vinifera* in order to provide a purified liquid extract of *Vitis vinifera;*
a6) optionally drying said extract of *Vitis vinifera* in order to provide a dried extract of *Vitis vinifera.*

Typically, the solvent is comprised in an amount 2 to 15 times, preferably 2 to 10 times of the weight of the raw material of Vinis vinifera.

Preferably, said step a2) is performed during 30 minutes to 24 hours.

Preferably, said step a2) is performed at a temperature ranging from 20°C to 100°C, preferably from 20°C to 80°C.

Preferably, said step a3) is performed by filtration, centrifugal decantation or by pressing, preferably by filtration.

Preferably, said step a4) is performed by evaporation under vacuum. Typically, said step is performed at a temperature inferior to 60°C, for example at 50°C. Typically, said step is performed at a pressure inferior to 100 mbars, for example at 60 mbars.

Preferably, said step a5) eliminates oligomers and polymers having a degree of polymerization superior to 12, preferably superior to 10. In other words, the extract of *Vitis vinifera* obtained by the purification step consists essentially of monomers and oligomers having a degree of polymerization between 2 and 12, preferably between 2 and 10, which are highly bioavailable compared to a non-purified extract of *Vitis vinifera.* In a particular embodiment, said step a5) comprises the steps of:

- filtrating said liquid extract of *Vitis vinifera,* preferably at a threshold of 15000 daltons, more preferably of 5000 daltons, in order to provide a filtrated liquid extract of *Vitis vinifera* and a retentate (to be discarded);
- optionally purifying said filtrated liquid extract of *Vitis vinifera* with a resin in order to provide a purified liquid extract of *Vitis vinifera.*

The skilled person is able to determine the parameters of the step of purifying the filtrated liquid extract of *Vitis vinifera* with a resin and can refers to the Examples.

Preferably, said step a6) is performed by spray-drying or sublimation, preferably by spray-drying. Typically, a drying carrier is used such as a maltodextrin.

In a particular embodiment, step b) comprises the steps consisting of:

b1) providing a raw material of *Vaccinium angustifolium,* preferably consisting of leafs and fruits of *Vaccinium angustifolium,*

b2) adding said raw material of *Vaccinium angustifolium* in a solvent, preferably an aqueous solution and/or ethanol, in order to provide a liquid slurry of *Vaccinium angustifolium,*

b3) separating the liquid fraction from said slurry of *Vaccinium angustifolium,* in order to provide a liquid solvent-containing extract of *Vaccinium angustifolium,* b4) eliminating the solvent from said liquid solvent-containing extract of *Vaccinium angustifolium,* in order to provide a liquid extract of *Vaccinium angustifolium,*

b5) optionally drying said extract of *Vaccinium angustifolium* in order to provide a dried extract of *Vaccinium angustifolium.*

Typically, the solvent is comprised in an amount 2 to 15 times, preferably 2 to 10 times of the weight of the raw material of *Vaccinium angustifolium.*

Preferably, said step b2) is performed during 30 minutes to 24 hours.

Preferably, said step b2) is performed at a temperature ranging from 20°C to 100°C, preferably from 20°C to 80°C.

Preferably, said step b3) is performed by filtration, centrifugal decantation or by pressing, preferably by filtration.

Preferably, said step b4) is performed by evaporation under vacuum. Typically, said step is performed at a temperature inferior to 60°C, for example at 50°C. Typically, said step is performed at a pressure inferior to 100 mbars, for example at 60 mbars.

Preferably, said step b5) is performed by spray-drying or sublimation, preferably by spray-drying. Typically, a drying carrier is used such as maltodextrin.

In a particular embodiment, said steps a) and b) are performed simultaneously by preparing an extract of *Vitis vinifera* and an extract of *Vaccinium angustifolium.* Said particular embodiment preferably comprises the steps consisting of:

ab1) providing a raw material of *Vitis vinifera,* preferably consisting of skins and seeds of *Vitis vinifera,*

ab2) providing a raw material of *Vaccinium angustifolium,* preferably consisting of leafs and fruits of *Vaccinium angustifolium,*

ab3) adding said raw material of *Vitis vinifera* and said raw material of *Vaccinium angustifolium* in a solvent, preferably an aqueous solution and/or ethanol, in order to provide a liquid slurry of *Vitis vinifera* and *Vaccinium angustifolium,*

ab4) separating the liquid fraction from said slurry of *Vitis vinifera* and *Vaccinium angustifolium,* in order to provide a liquid solvent-containing extract of *Vitis vinifera* and *Vaccinium angustifolium,*

ab5) eliminating the solvent from said liquid solvent-containing extract of *Vitis vinifera* and *Vaccinium angustifolium,* in order to provide a liquid extract of *Vitis vinifera* and *Vaccinium angustifolium,*

ab6) optionally drying said extract of *Vitis vinifera* and *Vaccinium angustifolium.*

The preferred above-mentioned embodiments, regarding for example optional steps (e.g., purification step), parameters and materials, can also be used in this particular embodiment.

Preferably, an antioxidant composition as defined herein is in a powder form.

According to a particular embodiment, an antioxidant composition as defined herein is added to a pet food preparation. The pet food may further comprise at least one pet food ingredient, preferably selected from the group consisting of proteins, peptides, amino acids, grains, carbohydrates, fats or lipids, nutrients, palatability enhancers, animal digests, meat meals, gluten, preservatives, surfactants, texturing or texturizing or stabilizing agents, colouring agents, inorganic phosphate compounds, flavours and/or seasonings.

In a particular embodiment, the pet food is a dog food, typically a kibble. The dog food may further comprise at least one pet food ingredient selected from the group consisting of ascorbic acid and/or vitamins, such as vitamin B1, and/or protein hydro lysates.

In a particular embodiment, the pet food is a cat food, typically a kibble. The cat food may further comprise at least one pet food ingredient selected from the group consisting of pyrophosphates, phyllosilicates, glucomannans and/or free amino acids. Preferably, said pyrophosphate is selected from the group consisting of sodium acid pyrophosphate, trisodium pyrophosphate, tetrasodium pyrophosphate, potassium acid pyrophosphate, tripotassium pyrophosphate and/or tetrapotassium pyrophosphate, more preferably trisodium pyrophosphate.

Another aspect of the present invention provides herein a method for preparing a pet food, comprising:

i) providing an antioxidant composition comprising an extract of *Vitis vinifera* and an extract of *Vaccinium angustifolium,* wherein said antioxidant composition comprises resveratrol, at least 1 % of catechins and/or epicatechins and 5 ppm of ferulic acid, wherein the extract of *Vitis vinifera* is obtained from at least the skins of *Vitis vinifera,* wherein said extract of *Vitis vinifera* provides said resveratrol, catechins and/or epicatechins, and wherein said extract of *Vaccinium angustifolium* provides said ferulic acid;
ii) providing a pet food preparation comprising at least one pet food ingredient;
iii) adding said antioxidant composition to said pet food preparation; and
iv) obtaining said pet food.

In particular, the antioxidant composition may be added to said pet food preparation at step iii) by coating or by inclusion, preferably by inclusion.

The products as described above, i.e. an antioxidant composition, a pet food and a pet supplement, can be used either in a therapeutic use, as a medicament, or as a non-therapeutic use.

The disclosure describes the non-therapeutic use of a synergistically-effective antioxidant composition or of a pet food or a pet supplement as above defined in a pet.

The disclosure thus describes such a use of a synergistically-effective antioxidant composition as above defined, for enhancing cognitive functions and/or executive functions in a healthy pet.

The term "enhancing cognitive functions and/or executive functions" has to be understood as facilitating, maintaining, or enhancing cognitive function and/or executive functions in the pet and/or as limiting non-pathological cognitive decline related to ages. The invention is thus useful for enhancing memory, attention, concentration, alertness, spatial orientation, following instructions, learning, intelligence, language, mood, stress, anxiety, vision and /or sleep. In a preferred embodiment, an antioxidant composition as defined herein is used for enhancing memory.

The present disclosure describes the therapeutic use of a synergistically-effective antioxidant composition or of a pet food or a pet supplement as above defined.

The disclosure thus describes a synergistically-effective antioxidant composition as above defined, for use as a medicament for pets.

More particularly, the disclosure describes a synergistically-effective antioxidant composition as above defined for use in a method for treating a neurodegenerative disorder in a pet.

A particular embodiment of the present disclosure relates to a pet food or pet supplement as above defined for use as a medicament for pets, especially for use in a method for treating a neurodegenerative disorder in a pet.

In other words, the disclosure describes a method for treating a neurodegenerative disorder in a pet, comprising administering to a pet in need thereof a therapeutically effective amount of:

- a synergistically-effective antioxidant composition as above defined; or
- at pet food or pet supplement as above defined.

Preferably, said neurodegenerative disorder is selected from the group consisting of Alzheimer's disease like disorders in pets, Parkinson disease, Huntington's disease, pathologic cognitive decline, schizophrenia, mild cognitive impairment, dementia, pre-dementia, diabetes, amnesia, mental retardation, memory disorder, dysthymia, involutive Depression and Confusional Syndrome. Preferably, the neurodegenerative disorder is a neurodegenerative disorder with cognitive deficiencies and/or executive deficiencies, preferably cognitive deficiencies. More preferably, the neurodegenerative disorder is selected from the group consisting of Alzheimer's disease like disorders in pets, memory disorder, dysthymia, Involutive Depression and Confusional Syndrome.

In a particular embodiment of the present disclosure, the neurodegenerative disorder is thus an Alzheimer's disease like disorders in pets, more preferably Cognitive Dysfunction Syndrome.

Indeed, it has been demonstrated in the present invention that an antioxidant composition as above defined acts in particular by synergistically enhancing the total antioxidant status in plasma of pets and significantly enhancing the working memory of pets. Indeed, the number of pets, such as dogs, having a good working memory is better when pets are fed with a pet food or a pet supplement according to the invention than without any treatment.

The skilled person is able to determine the appropriate amount of an antioxidant composition in a pet food or pet supplement, for example regarding the type, or the weight of pet food or pet supplement.

In a particular embodiment of the disclosure, a pet food or a pet supplement, preferably a kibble, comprises at least 50 ppm, preferably at least 100 ppm, more preferably 150 ppm, more preferably at least 200 ppm of an antioxidant composition of the invention.

In another particular embodiment of the disclosure, an effective amount of the antioxidant composition as defined herein is sufficient for achieving a therapeutic or prophylactic effect. For example, an antioxidant composition or a pet food or pet supplement may be administered so as to provide a daily amount equal to 0.1 mg/Kg or superior to 0.1 mg/kg and inferior to 50mg/kg or equal to 50mg/kg, for example about 0.5, 1 , 5, 10, 15, 20, 25, 30, 35, 40, 45 or 50 mg, per kilogram body weight of the patient, and it may be administered as a single daily dose, or divided into one or more daily doses, for example two or three daily doses. The compositions as defined herein can also be administered in combination with each other, or with one or more additional therapeutic or prophylactic compounds.

In a preferred embodiment, the antioxidant composition for use in pet food or pet supplement can provide to the pet a concentration of at least 100 μg/kgbw/day of catechins and/or epicatechins, preferably at least 150 μg/kgbw/day of catechins and/or epicatechins, more preferably 200 μg/kgbw/day of catechins and/or epicatechins, more preferably 220 ug/kgbw/day of catechins and/or epicatechins.

Preferably, the antioxidant composition for use in pet food or pet supplement can provide to the pet a concentration of at least 0.05 μg/kgbw/day of ferulic acid, preferably at least 0.06 μg/kgbw/day of ferulic acid, more preferably 0.07 μg/kgbw/day of ferulic acid, more preferably 0.08 μg/kgbw/day of ferulic acid.

Preferably, the antioxidant composition for use in pet food or pet supplement can provide to the pet a concentration of at least 10 μg/kgbw/day of resveratrol, preferably at least 15 μg/kgbw/day of resveratrol, more preferably 20 μg/kgbw/day of resveratrol, more preferably 21 μg/kgbw/day of resveratrol.

Preferably, the antioxidant composition for use in pet food or pet supplement can provide to the pet a concentration of at least 1.5 μg/kgbw/day of anthocyanins, preferably at least 1.7 μg/kgbw/day of anthocyanins, more preferably at least 2 μg/kgbw/day of anthocyanins, more preferably at least 2.5 μg/kgbw/day of anthocyanins, preferably at least 2.7 μg/kgbw/day of anthocyanins.

Preferably, the antioxidant composition for use in pet food or pet supplement can provide to the pet a concentration of at least 0.1 μg/kgbw/day of quercetin, preferably at least 0.2 μg/kgbw/day of quercetin, more preferably at least 0.3 μg/kgbw/day of quercetin, more preferably at least 0.37 μg/kgbw/day of quercetin.

The present disclosure also relates to a method for feeding pets comprising at least:

α) providing a pet food or pet supplement as above defined; and
β) feeding said pet food or pet supplement to pets.

Advantageously, said method enables one to treat a pet which suffers from a neurodegenerative disorder as above defined, and which is fed according to this method. Alternatively, said method enables one to non-therapeutically enhance cognitive functions and/or executive functions of a pet which is fed according to this method.

Another aspect of the present invention concerns a kit comprising, in one or more containers in a single package:

a) one or more pet food ingredient, preferably selected from the group consisting of proteins, peptides, amino acids, grains, carbohydrates, fats or lipids, nutrients, palatability enhancers, animal digests, meat meals, gluten, preservatives, surfactants, texturing or texturizing or stabilizing agents, colouring agents, inorganic phosphate compounds, flavours and/or seasonings; and
b) a (synergistically-effective) antioxidant composition according to the present invention.

Particular kits according to the present disclosure further comprise a means for communicating information or instructions, to help using the kits' elements.

The present disclosure also relates to at least:

a) one or more pet food ingredient, preferably selected from the group consisting of proteins, peptides, amino acids, grains, carbohydrates, fats or lipids, nutrients, palatability enhancers, animal digests, meat meals, gluten, preservatives, surfactants, texturing or texturizing or stabilizing agents, colouring agents, inorganic phosphate compounds, flavours and/or seasonings; and

b) a synergistically-effective antioxidant composition as above defined,

as a combined preparation for simultaneous, separate or sequential use in a method for treating a neurodegenerative disorder or for non-therapeutically enhancing cognitive functions and/or executive functions in a pet.

The present invention will be further described by reference to the following examples, which are presented for the purpose of illustration only and are not intended to limit the scope of the invention.

**EXAMPLES**

**Example 1: Process of preparation of an antioxidant composition of the invention**

Cakes (skins and seeds) of grape *Vitis vinifera* produced as the by-product of the wine industry and pomace of blueberries *Vaccinium angustifolium* produced from the juice industry were used as raw materials.

1000g of blueberry pomace were crushed and mixed with 5000ml 60%V/V ethanol solution with 0.1% wt HCl content. The mixture was kept at room temperature (20°C) during 24 hours. The ethanolic solution was then separated from the pomace thanks to filtration, and concentrated under vacuum with a rotary evaporator at 20% dry matter.

400g of cakes from *Vitis vinifera* were mixed with 1500ml 80%V/V ethanol solution at 60°C during 5 hours. The ethanolic solution was then separated from the pomace thanks to filtration. Ethanol was then removed under vacuum with a rotary evaporator at a temperature of 50°C under 60mbars. The aqueous solution was then diluted in order to obtain 5% of dry matter, and filtered on a 5000 daltons membrane. The permeate was loaded on a resin (C18) column at 1BV/hour. The resin was then first flushed with 3 BV of distilled water at 2BV/h, then eluted with 5BV of a 80% v/v ethanol solution at 1 BV/h. A part of the extracted solution is kept to be characterized (Table 1) and tested.

*Table 1 : Flavonols of Vitis vinifera extract*

| Concentration in monomers and oligoprocyanidins (% dry Weight - eq. Epicatechins) (measured by Liquid Chromatography- fluorescence detection) | |
|---|---|
| Monomers | 9.4% ± 0.8 |
| Dimers | 4.0% ± 0.3 |
| Trimers | 0.81% ± 0.1 |
| Tetramers | 0.24% ± 0.1 |
| Pentamers | Non detected |
| Hexamers | Non detected |
| Heptamers | Non detected |
| Octamers | Non detected |
| Nonamers | Non detected |
| Decamers | Non detected |

Both obtained extracts of *Vaccinium angustifolium,* and of *Vitis vinifera* were mixed together and a maltodextrin is added to this mix, in order to obtain 30% of dry matter. The resulting blend was then spray dried with an inlet temperature of 160°C.

The product obtained ("mix") was a free flowing purple powder containing different polyphenols as shown at table 2.

*Table 2: Polyphenols of the mix according to the example 1 of the invention (measured by UPLC-MS/MS)*

| | Grape extract *Vitis vinifera* | Blueberry extract *Vaccinium angustifolium* | Mix of *Vitis vinifera* and *Vaccinium angustifolium* |
|---|---|---|---|
| Catechins and epicatechins | X | - | 9.4% |
| Anthocyanins (malvidine-3-glucoside) | - | X | 700 ppm 328 ppm |

(continued)

| | Grape extract *Vitis vinifera* | Blueberry extract *Vaccinium angustifolium* | Mix of *Vitis vinifera* and *Vaccinium angustifolium* |
|---|---|---|---|
| Quercetin and quercetin glycosides | - | X | 77 ppm |
| Ferulic acid | - | X | 20 ppm |
| Resveratrol | X | - | 5327 ppm |
| X= Presence in the extract | | | |

**Example 2: Effect of treatment of dogs with an antioxidant composition of the invention on Total Antioxidant Status.**

The aim of this assay was to check the efficacy of a mix containing both grape extract (*Vitis vinifera*) and blueberry extracts (*Vaccinium angustifolium*), as obtained in Example 1, to improve antioxidant status of adult dogs by comparing them to dogs receiving either grape extract as itself, blueberry extract as itself or a placebo (control).

2.1. Material and methods

Nine Beagle dogs (6 males and 3 females, Body Condition Score (BCS) 5/9, mean age 20 $\pm$ 0.9 months, mean body weight 9.1 $\pm$ 0.4 kg) were fed with a maintenance diet to maintain their body weight. Dogs were consecutively supplemented with pet supplements, i.e. capsules of gelatin (Cooper, Melun Cedex, France) containing either maltodextrin (placebo as a Control; Glucidex12 lot#421A323532, Roquette, Lestrem Cedex, France), grape extract (Grape; Neurogrape Inside PC PR120 batchAI50288, Activ'Inside, Libourne, France), Blueberry extract (Wild blueberry extract 0.4TP lot#294, Nutra Canada, Québec, Canada), or "mix", i.e. a composition of the invention as obtained in Example 1 (grape and blueberry extracts; 4 mg/kg BW/day). The amount of compounds provided by this diet is presented at table 3.

*Table 3*

| | **Mix** | **4mg of mix/kg of body weight (bw)** |
|---|---|---|
| Catechin and epicatechin | 9.4% | 220 $\mu$g/kgbw/day |
| Anthocyanins | 700ppm | 2.8 $\mu$g/kgbw/day |
| Quercetin and quercetin glycosides | 77ppm | 0.37$\mu$g/kgbw/day |
| Ferulic acid | 20ppm | 0.08 $\mu$g/kgbw/day |
| Resveratrol | 5327ppm | 21 $\mu$g/kgbw/day |

The experiment was designed as a crossover study where dogs received the capsule supplementation during 28 days with a week of wash out among each supplementation period. Each dog thus received each supplementation.

Blood samples were collected from the jugular vein before and after each supplementation, and were kept on ice. Plasma was recovered by centrifuging whole blood at 2124G during 10 min at 4°C. Aliquots of plasma were incubated at -80°C.

Oxidant status was evaluated by measuring Total Antioxidant Status (TAS). To this end, a colorimetric-based assay available from RANDOX laboratories (Ref: NX2332, Crumlin, County Antrim, UK) was used to evaluate the total antioxidant status. This methodology involves incubating 2,2'-Azino-di-[3-ethylbenzthiazoline sulphonate] (ABTS) with a peroxidase (metmyoglobin) and $H_2O_2$ to produce the radical cation ABTS$^+$. This has a relatively stable blue-green color, which is measured at 600nm. Antioxidants in the added sample cause suppression of this color production to a degree which is proportional to their concentration. The Total Antioxidant Status (TAS) was expressed as mmol/L.

In order to compare the four diets with each others, the $\Delta$TAS was determined by comparing TAS before and after supplementation: $\Delta$TAS = TAS Day 28 - TAS Baseline.

$\Delta$TAS was analyzed using one mixed-effect model. This model includes the fixed categorical effects of baseline, treatment and order of randomization. This model was run using SAS (v9.4) mixed procedure with an unstructured correlation matrix to model the within-animal errors. Parameters were estimated using restricted maximum likelihood method with the Newton-Raphson algorithm. Denominator degrees of freedom were estimated using Satterthwaite's approximation. All effects were evaluated at the $\alpha$= 0.10 level.

The Wilcoxon test was used to compare changes in TAS before and after supplementation. Alpha level for determination of significance was 0.10.

## 2.2. Results

The results regarding the total antioxidant status obtained with the different supplementations (Mean and S.E (standard error)) are presented in Table 3 hereafter. The results regarding the Wilcoxon test are also reported in Table 4.

*Table 4: TAS (mmol/L) (Mean $\pm$ SEM) taken from 4 groups of adult dogs (n = 9) feeding with a experimental diet supplemented with the Mix, grape extract (Gra), blueberry extract (Blu) or maltodextrin (Control) during 28 days.*

|  |  | Day 0 | | Day 28 | | p-value (Wilcoxon) |
|---|---|---|---|---|---|---|
|  |  | Mean | S.E. | Mean | S.E. | Treatment |
| TAS mmol/L | **Blu** | 0.92 | 0.122 | 0.90 | 0.109 | NS |
|  | **Mix** | 0.81 | 0.052 | 0.93 | 0.070 | 0.023 |
|  | **Gra** | 0.97 | 0.155 | 0.87 | 0.077 | NS |
|  | **Control** | 0.84 | 0.048 | 0.88 | 0.075 | NS |
| *NS : not significant. | | | | | | |

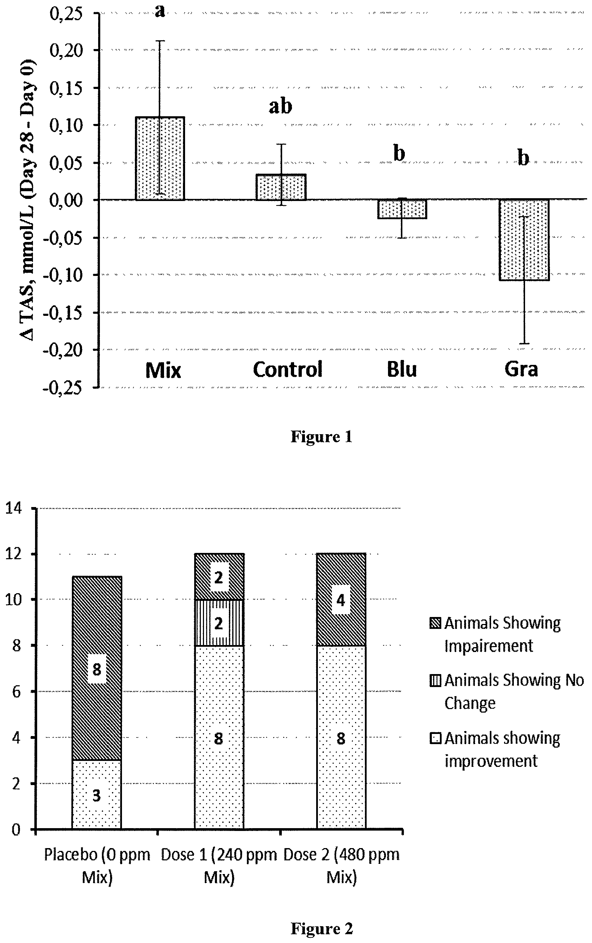

As shown in Table 4 and Figure 1, the grape and blueberry extracts contained into the Mix improved significantly and synergistically the TAS concentration compared to the grape extract and the blueberry extract taken alone.

Moreover, the results of the Wilcoxon test show that the mix (both grape and blueberry extracts) is the only supplementation which presented significantly higher TAS concentrations after supplementation. Supplementations with grape extract or blueberry extract alone did not significantly changed TAS concentration.

Dog's supplementation with a mix containing both grape and blueberry extracts has thus a synergistic effect on the total antioxidant status of the animal compared to supplementations with blueberry and grape extracts taken alone.

**Example 3: Effect of treatment with an antioxidant composition of the invention on dog's memory.**

The aim of this assay was to check the effect of a mix according to the invention containing grape and blueberry extracts, as obtained in Example 1, at two dose levels on working memory in dogs.

### 3.1. Material and methods

The study was a randomized and blinded study, where a longitudinal parallel group design was used. Thirty-five (35) Beagle dogs (Vivocore Inc. colony, Toronto, Canada; 14 males and 21 females; ages ranged from 8.0 to 14.5 years at study initiation) were allocated in 3 groups three weeks in advance of experimental diets supplementation. This allocation was based on performance (cumulative scores) on the baseline delayed non-matching position (DNMP).

Following group allocation, 3 groups of dogs were then respectively fed during 8 weeks with pet foods, i.e. kibbles containing either 0 ppm (control), 240 ppm of mix or 480 ppm of mix.

Nutritionally-balanced dry dog foods suitable for consumption by dogs and obtained after an extrusion and drying process, were prepared, herein referred to as "XX". The formulation was exactly the same, with the exception of two incorporating either 240 ppm or 480 ppm of mix (ppm based on the kibble weight) as obtained in Example 1 in their formulation before extrusion. The mix was thus added to the pet food preparation by inclusion.

The diets were herein referred as "Control" for the diet without mix, i.e. without grape and blueberry extracts, added in the formulation, and "Dose 1" (including 240 ppm mix) and "Dose 2" (including 480 ppm mix) for the diets including an antioxidant composition according to the invention in the formulation. All diets underwent extrusion and drying process. A palatability enhancer (2%; chicken digest) was added on the kibbles, i.e. by coating at the end of the process.

DNMP (Delayed Non-Matching to Position) testing (6,7) was carried out on days - 27 to -16 of baseline, and on study days 58 through 63. Testing was performed according to standard operating procedure.

DNMP test comprises two phase test:

- Sample phase: dog is required to displace an object placed over 1 of 3 possible positions on a food well. The block to be displaced covers a food reward;

- Second stage: after a delay (20s vs 90s), the dog is presented with 2 objects identical to the sample phase. An object is located at the same position as for the sample phase. However the correct object is placed in one of the 2 remaining positions (non match), if the dog displaces this object, he gets the food reward.

There were 12 trials per DNMP test session and there was one test session per day for all subjects. The variable delay subtask was used. For each test session, delays of 20 and 90 seconds were equally divided among the 12 trials, allowing for the assessment of working memory. An inter-trial interval of 30 seconds was used.

In the present study, six sessions were used for the baseline phase and six sessions for the treatment phase. Subjects were tested on each of the designated days regardless of score.

During all testing procedures, animals were rewarded with Purina Essential Care Adult Formula wet canned dog food.

In order to compare the three treatments on DNMP results, the improvements over baseline were analyzed using a Chi square test. This analysis was run using SAS (v9.4) freq procedure with significance level of $\alpha = 0.05$.

3.2. Results

As shown in figure 2, dogs which were treated with a mix of the invention showed significantly improved cognitive functions, in particular memory, compared to dogs which did not receive any treatment.

Moreover, the amount of treatment did not affect the number of dogs with significative improvements, which demonstrates the effiency of the treatment according to the invention irrespective of the dose of treatment.

**Example 4: Effect on palatability of an antioxidant composition added to a dry pet food.**

The examples below report palatability assessments of different pet food products (cats or dogs), coated with various palatability enhancers and comprising various amounts of an antioxidant composition of the invention.

4.1. Material and methods

*4.1.1. Method of palatability assessment: the "two-bowl" test*

*Principle of the two-bowl test:*

The test is based on the postulate whereby the more food consumed, the more palatable it is.

Individual versus (Two bowls) appetence tests, based on the comparison between two foods, were carried out.

*Operating method of the test:*

- Identical amounts of food product A and food product B were weighed out and placed in identical bowls. The amount present in each ration enables the daily requirements to be met.

- Distribution of the bowls:

\* Dog test: the bowls were placed in an individual feed trough accessible to dogs.

\* Cat test: The bowls were presented at the same time to each cat in an individual loose box and their positions were switched at each meal to avoid a choice led by handedness.

- Duration of the test:

\* Cat test for dry food: from about 15 minutes to about 20 hours (if one of the two bowls was entirely eaten before the end of the test, the two bowls were removed, and the test was stopped);

\* Dog test for dry food: from about 15 minutes to about 30 minutes (if one of the two bowls was entirely eaten before the end of the test, the two bowls were removed, and the test was stopped).

- Measured parameters:

First food consumed ("attractiveness") and amount of each food consumed by the end of the test;

- Calculated parameters:

Individual consumption ratio in % (CR)

$$CRA = \text{consumption of A (g) x } 100/(\text{consumption of A+B) (g)}$$

$$CRB = \text{consumption of B (g) x } 100/(\text{consumption of A+B) (g)}$$

→ Average consumption ratio (ACR) = average of all individual ratios (an equal importance is given to each animal, regardless of its size and of its corresponding consumption).

If animals have higher or lower consumption compared to predetermined values (which are function of, e.g., the animal weight and/or metabolism), they are not taken into account into statistical treatment.

*Statistical analysis:*

Statistical analysis was used to determine if there was a significant difference between the 2 ratios. A Student's t-test with 3 error thresholds, namely 5%, 1% and 0.1%, was performed.

A Chi-square test was used to determine if there was a significant difference between the number of pets with Food A as first food eaten and the number of pets with Food B as first food eaten.

Significance levels are noted as below:

| NS | not significant | ($p > 0.05$) |
|---|---|---|
| \* | significant | ($p \leq 0.05$) |
| \*\* | highly significant | ($p \leq 0.01$) |
| \*\*\* | very highly significant | ($p \leq 0.001$) |

*4.1.2. Diets*

Control and experimental diets were prepared by using nutritionally-complete foods that contained all known required nutrients for the intended consumer of the food, in appropriate amounts and proportions based, for example, on recommandations of recognized or competent authorities in the field of companion animal nutrition. Such foods are therfore capable of serving as a sole source of dietary intake to meet essential needs of pets.

When the ingredients to be tested were applied topically on a dry food by coating liquid and/or dry ingredients, a pet food composition was used in the form of "uncoated kibbles". That was a partially-ended food, on which, then, were coated fat and palatability enhancers.

When the ingredients to be tested were applied by inclusion, those ingredients were mixed with other ingredients before extrusion (for dry or semi dry pet foods) or before sterilization (for wet pet foods).

When the ingredients to be tested were applied in drinkable compositions, the ingredients were mixed with water or with the fluid compositions.

4.2. Results

4.2.1. Example 4A: Effect on palatability of 0.020% of antioxidant composition added to a dry dog food by coating.

A nutritionally-balanced dry kibble suitable for consumption by dogs and obtained after an extrusion and drying process, was prepared, herein referred to as "VV".

In this example, control diet A was "VV" coated with 6% of poultry fat and 1.5% of palatability enhancer PE1 in powder (which is a pork digest A).

Experimental diet B was "VV" coated with 6% of poultry fat, 1.5% of PE1 and 0.02% of "mix", i.e. the antioxidant composition obtained in Example 1 (% by weight of dry kibble VV). Kibbles were first coated with poultry fat, and further coated with a previously prepared composition containing PE1 and the mix.

As shown in Table 5 below, consumption of the food was not significantly different between Control diet A and Experimental Diet B, demonstrating the neutral effect on palatability for dog of the experimental diets coated with 0.02% of the mix, i.e. an antioxidant composition of the invention.

*Table 5*

| Food control ("A" for analysis) | Food experimental ("B" for analysis) | 1st choice & stat. Significance | Consumption ratio | | Statistical significance | Number of validated animals |
|---|---|---|---|---|---|---|
| | | | %A | %B | | |
| Control Diet A (1.5% PE1 without mix) | Expe Diet B (1.5% PE1 and 0.02% of mix by coating) | B NS | 46 | 54 | NS | 31 |

4.2.2. Example 4B: Effect on palatability of 0.1% of antioxidant composition added to a dry dog food by inclusion.

A nutritionally-balanced dry kibble suitable for consumption by dogs and obtained after an extrusion and drying process, was prepared, herein referred to as "YY".

The formulation was exactly the same between the control diet A and the experimental diet B, with the exception of the experimental diet B incorporating 0.1% of mix by weight of kibble in the formulation before extrusion. The mix was thus added to the pet food preparation by inclusion. Kibbles were then coated with 6% of poultry fat and 3% of palatability enhancer (1% of PE1 and 2% of another liquid palatability enhancer PE2, which is a pork digest B).

As shown in Table 6 below, consumption of the food was not significantly different between Control diet A and Experimental Diet B, demonstrating the neutral effect on palatability for dog of the experimental diets including 0.1% of the mix, i.e. an antioxidant composition of the invention.

*Table 6*

| Food control ("A" for analysis) | Food experimental ("B" for analysis) | 1st choice & stat. Significance | Consumption ratio | | Statistical significance | Number of validated animals |
|---|---|---|---|---|---|---|
| | | | %A | %B | | |
| Control Diet A (1% PE1 and 2% PE2 without mix) | Expe Diet B (1% PE1, 2% PE2 by coating and 0.1% of mix by inclusion) | B NS | 53 | 47 | NS | 33 |

4.2.3. Example 4C: Effect on palatability of 0.024% of antioxidant composition added to a dry dog food by inclusion.

A nutritionally-balanced dry kibble suitable for consumption by dogs and obtained after an extrusion and drying process, was prepared, herein referred to as "NN".

The formulation was exactly the same between the control diet A and the experimental diet B, with the exception of the experimental diet B incorporating 0.024% of mix by weight of kibble in the formulation before extrusion. The mix and a palatability enhancer (1% of PE3, dry palatability enhancer, which is a pork digest C) were thus added to the pet food preparation by inclusion. Kibbles were then coated with 8% of poultry fat and 2.5% of palatability enhancer (palatability enhancer PE4 in a liquid form, which is a pork digest D).

As shown in Table 7 below, consumption of the food was not significantly different between Control diet A and Experimental Diet B, demonstrating the neutral effect on palatability for dog of the experimental diets including 0.024% of the mix, i.e. an antioxidant composition of the invention.

*Table 7*

| Food control ("A" for analysis) | Food experimental ("B" for analysis) | 1st choice & stat. Significance | Consumption ratio | | Statistical significance | Number of validated animals |
|---|---|---|---|---|---|---|
| | | | %A | %B | | |
| Control Diet A (1% PE3 by inclusion and 2.5% PE4 without mix by coating) | Expe Diet B (2.5% PE4 by coating and 1% PE3 and 0.024% of mix by inclusion) | B NS | 52 | 48 | NS | 31 |

4.2.4. Example 4D: Effect on palatability of 0.02% of antioxidant composition added to a dry cat food by coating.

A nutritionally-balanced dry kibble suitable for consumption by cats and obtained after an extrusion and drying process, was prepared, herein referred to as "ZZ".

In this example, control diet A was "ZZ" coated with 6% of poultry fat and 1.5% of palatability enhancer PE5 in powder (which is a pork digest E).

Experimental diet B was "ZZ" coated with 6% of poultry fat, 1.5% of PE5 and 0.02% of "mix", i.e. the antioxidant composition obtained in Example 1 (% by weight of dry kibble VV). Kibbles were first coated with poultry fat, and further coated with a previously prepared composition containing PE5 and the mix.

As shown in Table 8 below, consumption of the food was not significantly different between Control diet A and Experimental Diet B, demonstrating the neutral effect on palatability for cats of the experimental diets coated with 0.02% of the mix, i.e. an antioxidant composition of the invention.

*Table 8*

| Food control ("A" for analysis) | Food experimental ("B" for analysis) | 1st choice & stat. Significance | Consumption ratio | | Statistical significance | Number of validated animals |
|---|---|---|---|---|---|---|
| | | | %A | %B | | |
| Control Diet A (1.5% PE5 without mix) | Expe Diet B (1.5% PE5 and 0.02% of mix by coating) | B NS | 48 | 52 | NS | 39 |

4.2.5. Example 4E: Effect on palatability of 0.1% of antioxidant composition added to a dry cat food by inclusion.

A nutritionally-balanced dry kibble suitable for consumption by cats and obtained after an extrusion and drying process, was prepared, herein referred to as "WW".

The formulation was exactly the same between the control diet A and the experimental diet B, with the exception of the experimental diet B incorporating 0.1% of mix by weight of kibble in the formulation before extrusion. The mix was thus added to the pet food preparation by inclusion. Kibbles were then coated with 6% of poultry fat and 1.5% of palatability enhancer PE5.

As shown in Table 9 below, consumption of the food was not significantly different between Control diet A and Experimental Diet B, demonstrating the neutral effect on palatability for cats of the experimental diets including 0.1% of the mix, i.e. an antioxidant composition of the invention.

*Table 9*

| Food control ("A" for analysis) | Food experimental ("B" for analysis) | 1st choice & stat. Significance | Consumption ratio | | Statistical significance | Number of validated animals |
|---|---|---|---|---|---|---|
| | | | %A | %B | | |
| Control Diet A (1.5% PE5 without mix) | Expe Diet B (1.5% PE5 and 0.1% of mix by inclusion) | A NS | 44 | 56 | NS | 35 |

**Example 5: Effect of treatment of dogs with an antioxidant composition of the invention included in a pet supplement or in a pet food on kinetics of absorption.**

The aim of this study was to compare the efficacy of two different modes of administration of an antioxidant composition according to the invention ("mix") in dogs. For this purpose, the kinetics of mix absorption for pet supplement, i.e. capsule administration or pet food inclusion in kibbles by extrusion process were compared, on the criteria of specific blood biomarkers of grape and blueberry polyphenols (also called phenolic biomarkers).

5.1.Material and methods

Six adult Beagle dogs (5 females and 1 male, age ranged from 5 to 8 years old) were fed a control diet (no polyphenol supplementation) to maintain their body weight during a 7 day wash out period. After this period, dogs received 240 ppm of the mix either in pet supplement, capsules (C), or directly in the pet food diet (D) during a 7 day wash in period (4 mg/kg BW). On the 8th day of supplementation, a blood collection was realized during 8 hours to evaluate the mix kinetics of absorption. For each kinetic, blood samples were taken 1h prior the ingestion of the mix and then at 0.5, 1, 2, 4 and 8h post-ingestion.
The experiment was designed as a crossover design where dogs received the same supplementation of mix, C or D, during 7 days with a week of wash out among each week of administration.
Polyphenol metabolites (or phenolic blood compounds) were extracted from plasma and eluted according to a specific protocol as described by Marti et al. (2011). The eluted solutions were directly injected in UHPLC-MS/MS for the analysis of the specific phenolic biomarkers of grape and blueberry polyphenols.
Each metabolite was analyzed using one mixed-effect model. This model includes the fixed categorical effects of Mode of administration (C or D), Time of blood collection, Sequence as well as administration x Time of blood collection interaction. This model was run using SAS (v9.4) mixed procedure with an unstructured correlation matrix to model the within-Animal (Sequence) errors. Parameters were estimated using restricted maximum likelihood method with the Newton-Raphson algorithm. Denominator degrees of freedom were estimated using Satterthwaite's approximation. All effects were evaluated at the $\alpha$ = 0.05 level.

5.2. Results

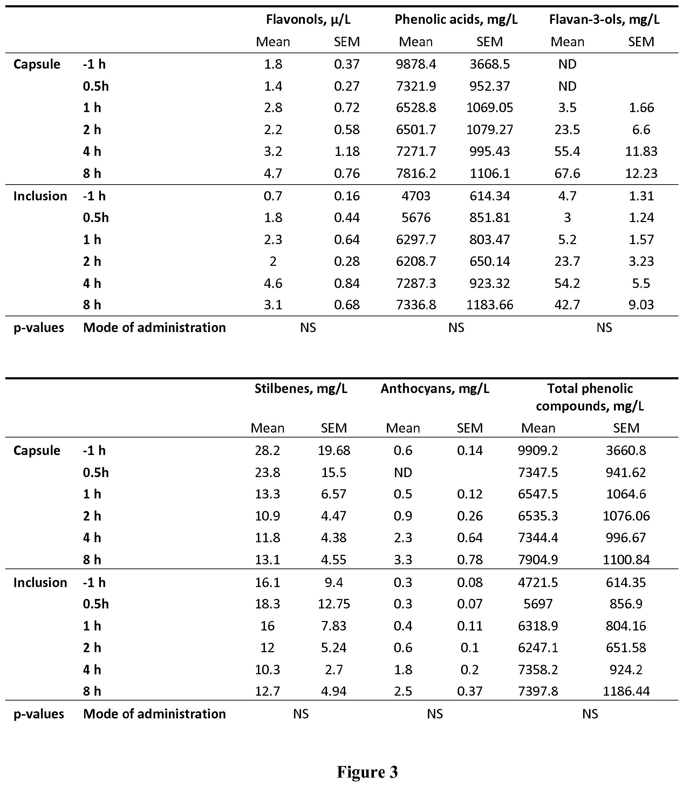

As summarized in Figure 3, there was no effect of the mode of administration on the mix metabolites dosed in the blood, whatever the time of blood collection..
At a dosage of 4 mg/kg BW, polyphenol metabolites could be analyzed in dog blood. These phenolic compounds were specific from the extracts present in the mix, i.e. either from grape origin (extract of *Vitis vinifera*) for the flavan-3-ols and silbenes phenolic families or from blueberry origin (extract of *Vaccinium angustifolium*) for flavanol, anthocyanin and phenolic acids families. These results indicate that the mix can be as efficiently absorbed when delivered by pet supplement or when directly included in the pet food.

**Example 6: Effect of treatment of dogs with an antioxidant composition of the invention on tolerance.**

The aim of this study was to check the safety of an antioxidant composition according to the invention ("mix") by monitoring early and specific renal and routine biomarkers in adult dogs.

6.1. Material and methods

Twenty four Beagle dogs (20 males and 4 females, BCS 5/9, mean age 31 $\pm$ 3 months, mean body weight 11.4 $\pm$ 0.2 kg), were fed a maintenance commercial diet (Royal Canin medium adult, France) to maintain their optimal body weight during the study. Four groups of 6 dogs each were supplemented with pet supplement, i.e. capsules containing maltodextrin (Placebo as a Control) or with an antioxidant composition of the invention, "mix" at 4 (Mix 1), 20 (Mix 5), 40 (Mix 10) mg/kg BW/day.
Blood and urine samples were taken at the beginning of the study (week 0), after 12 and after 24 weeks (week 12 and week 24).
Plasma and urinary cystatine C (CysC), clusterin and neutrophil gelatinase-associated lipocalin (NGAL) were analyzed in blood samples (8,9).
Each safety parameter was analyzed using one mixed-effect model. This model includes the fixed categorical effects of baseline, day, treatment as well as day x treatment interaction. This model was run using SAS (v9.4) mixed procedure with an unstructured correlation matrix to model the within-animal errors. Parameters were estimated using restricted maximum likelihood method with the Newton-Raphson algorithm. Denominator degrees of freedom were estimated using Satterthwaite's approximation. All effects were evaluated at the $\alpha = 0.05$ level.

6.2. Results

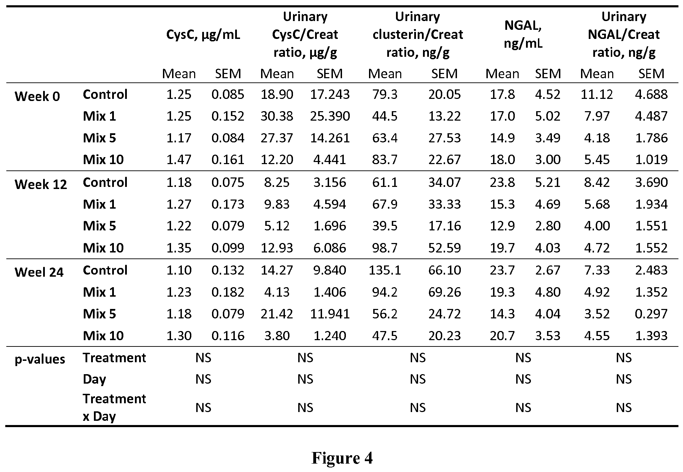

Early and specific renal biomarkers are exposed in Figure 4. These biomarkers were lower than our in-house upper limit obtained in control and experimental dogs at week 0. These in-house upper limit were 2.23 $\mu$g/mL, 156 ng/g, 443 ng/g, 47 ng/mL, 28.5 ng/g for plasma CysC, urinary CysC/Crea ratio, urinary clusterin/Creat ratio, plasma NGAL, urinary NGAL/Creat, respectively.

Adult dogs can safely consume a antioxidant composition as defined herein, containing an extract of *Vitis vinifera* (grape) and an extract of *Vaccinium angustifolium* (blueberry) given at supplemented doses, ranging from 4 to 40 mg/ kg BW/day. There was not any clinical sign of intolerance, even at the highest tested dose (10 times the normal dose).

**REFERENCES**

(1) London E D, Ohata M, Takei H et al (1983). Regional cerebral metabolic rate for glucose in beagle dogs of different ages, Neurobiol Aging 4: 121-126.)

(2) Maria Monagas et al, Commercial dietary ingredients from Vitis vinifera L. leaves and grape skins: antioxidant and chemical characterization, J. Agric. Food Chem. 2006, 54, 2139-327,

(3) Jayaprakasha et al., Antioxidant activity of grape seed (vitis vinifera) extracts on peroxidation models in vitro, Food chemistry 73 (2001) 285-290

(4) Eubig P, Brady M, Gwaltney-Brant S, Khan S, Mazzaferro E, Morrow C (2005). "Acute renal failure in dogs after the ingestion of grapes or raisins: a retrospective evaluation of 43 dogs (1992-2002)

(5) Cotman CW, Berchtold NC. Exercise: a behavioral intervention to enhance brain health and plasticity. Trends Neurosci. 2002;25(6):295-301

(6) Studzinski CM, Christie L-A, Araujo JA et al. Visuospatial function in the beagle dog: An early marker of cognitive decline in a model of human cognitive aging and dementia. Neurobiol Learn Mem 2006;86(2):197-204.

(7) Landsberg GM1, Nichol J, Araujo JA., 2012. Cognitive dysfunction syndrome: a disease of canine and feline brain aging. Vet Clin North Am Small Anim Pract. 2012 Jul;42(4):749-68, vii. doi: 10.1016/j.cvsm.2012.04.003. Epub 2012 May 17.

(8) Tvarijonaviciute A, Ceron JJ, Holden SL, Biourge V, Morris PJ, German AJ. Effect of Weight Loss in Obese Dogs on Indicators of Renal Function or Disease. J. Vet. Intern. Med. 2013;27:31-38.

(9) Garcia-Martinez JD, Tvarijonaviciute A, Ceron JJ, Calden M, martinez-Subierla S. Urinary Clusterin as a Renal Marker in Dogs. J. Vet. Diagn. Invest. 2012;24:301-306

**Claims**

**1.** An antioxidant composition for use in pet food or pet supplement comprising an extract of *Vitis vinifera* and an extract of *Vaccinium angustifolium,* wherein said antioxidant composition comprises resveratrol, at least 1 % of catechins and/or epicatechins, and at least 5 ppm of ferulic acid,
wherein the extract of *Vitis vinifera* is obtained from at least the skins of *Vitis vinifera,*
wherein said extract of *Vitis vinifera* provides said resveratrol, catechins and/or epicatechins, and
wherein said extract of *Vaccinium angustifolium* provides said ferulic acid.

**2.** The antioxidant composition according to claim 1 which provides to the pet a concentration of at least 100 $\mu$g/kgbw/day of catechins and/or epicatechins, preferably at least 150 $\mu$g/kgbw/day of catechins and/or epicatechins, more preferably 200 $\mu$g/kgbw/day of catechins and/or epicatechins, more preferably 220 $\mu$g/kgbw/day of catechins and/or epicatechins.

**3.** The antioxidant composition according to claim 1 or 2, which provides to the pet a concentration of at least 0.05 $\mu$g/kgbw/day of ferulic acid, preferably at least 0.06 $\mu$g/kgbw/day of ferulic acid, more preferably 0.07 $\mu$g/kgbw/day of ferulic acid, more preferably 0.08 $\mu$g/kgbw/day of ferulic acid.

**4.** The antioxidant composition according to any one of claims 1 to 3, wherein said antioxidant composition comprises at least 4000 ppm of resveratrol, preferably at least 4500 ppm of resveratrol, more preferably at least 5000 ppm of resveratrol, more preferably at least 5100 ppm of resveratrol, more preferably at least 5200 ppm of resveratrol, more preferably at least 5300 ppm of resveratrol.

5. The antioxidant composition according to any one of claims 1 to 4, which provides to the pet a concentration of at least 10 $\mu$g/kgbw/day of resveratrol, preferably at least 15 $\mu$g/kgbw/day of resveratrol, more preferably 20 $\mu$g/kgbw/day of resveratrol, more preferably 21 $\mu$g/kgbw/day of resveratrol.

6. The antioxidant composition according to any one of claims 1 to 5, wherein the ratio A/B, wherein A is resveratrol and B is catechins and/or epicatechins, is of at least 0.005, preferably at least 0.01, more preferably at least 0.02, more preferably at least 0.03, more preferably at least 0.04, more preferably at least 0.05, more preferably at least 0.06, more preferably at least 0.07, more preferably at least 0.08, more preferably at least 0.09.

7. The antioxidant composition according to any one of claims 1 to 6, wherein said antioxidant composition comprises at least 500 ppm of anthocyanins, preferably at least 600 ppm of anthocyanins, more preferably at least 650 ppm of anthocyanins, more preferably at least 700 ppm of anthocyanins.

8. The antioxidant composition according to any one of claims 1 to 7, which provides to the pet a concentration of at least 1.5 $\mu$g/kgbw/day of anthocyanins, preferably at least 1.7 $\mu$g/kgbw/day of anthocyanins, more preferably at least 2 ug/kgbw/day of anthocyanins, more preferably at least 2.5 $\mu$g/kgbw/day of anthocyanins, preferably at least 2.7 $\mu$g/kgbw/day of anthocyanins.

9. The antioxidant composition according to any one of claims 1 to 8, wherein said antioxidant composition comprises at least 50 ppm of quercetin, preferably 60 ppm of quercetin, more preferably 65 ppm of quercetin, more preferably 70 ppm of quercetin, more preferably 75 ppm of quercetin, more preferably 77 ppm of quercetin.

10. The antioxidant composition according to any one of claims 1 to 9, which provides to the pet a concentration of at least 0.1 $\mu$g/kgbw/day of quercetin, preferably at least 0.2 $\mu$g/kgbw/day of quercetin, more preferably at least 0.3 $\mu$g/kgbw/day of quercetin, more preferably at least 0.37 $\mu$g/kgbw/day of quercetin.

11. The antioxidant composition according to any one of claims 1 to 10 for use in a pet food, wherein said pet food further comprises at least one pet food ingredient, preferably selected from the group consisting of proteins, peptides, amino acids, grains, carbohydrates, fats or lipids, nutrients, palatability enhancers, animal digests, meat meals, gluten, preservatives, surfactants, texturing or texturizing or stabilizing agents, colouring agents, inorganic phosphate compounds, flavours and/or seasonings.

12. A method for preparing a pet food, comprising:

    i) providing an antioxidant composition according to any one of claims 1 to 10;
    ii) providing a pet food preparation comprising at least one pet food ingredient;
    iii) adding said antioxidant composition to said pet food preparation; and
    iv) obtaining said pet food.

13. The method according to claim 12, wherein said antioxidant composition is added to said pet food preparation at step iii) by coating or by inclusion, preferably by inclusion.

14. A kit comprising, in one or more containers in a single package:

    a) one or more pet food ingredients, preferably selected from the group consisting of proteins, peptides, amino acids, grains, carbohydrates, fats or lipids, nutrients, palatability enhancers, animal digests, meat meals, gluten, preservatives, surfactants, texturing or texturizing or stabilizing agents, colouring agents, inorganic phosphate compounds, flavours and/or seasonings; and
    b) an antioxidant composition according to any one of claims 1 to 10.

**Patentansprüche**

1. Antioxidans-Zusammensetzung zur Verwendung in Haustiernahrung oder Haustier-Nahrungsergänzungsmitteln, die einen Extrakt von *Vitis vinifera* und einen Extrakt von *Vaccinium angustifolium* enthält, wobei die Antioxidans-Zusammensetzung Resveratrol, mindestens 1 % Katechine und/oder Epikatechine und mindestens 5 ppm Ferulasäure enthält,
wobei der Extrakt von *Vitis vinifera* zumindest aus den Schalen der *Vitis vinifera* gewonnen wird, wobei der Extrakt von *Vitis vinifera* das Resveratrol, Katechine und/oder Epikatechine liefert, und
wobei der Extrakt von *Vaccinium angustifolium* die Ferulasäure liefert.

2. Antioxidans-Zusammensetzung nach Anspruch 1, die für das Haustier eine Konzentration von mindestens 100 μg/kg Körpergewicht/Tag von Katechinen und/oder Epikatechinen liefert, vorzugsweise mindestens 150 μg/kg Körpergewicht/Tag von Katechinen und/oder Epikatechinen, besser von 200 μg/kg Körpergewicht/Tag von Katechinen und/oder Epikatechinen, noch besser 220 μg/kg Körpergewicht/Tag von Katechinen und/oder Epikatechinen.

3. Antioxidans-Zusammensetzung nach Anspruch 1 oder 2, die für das Haustier eine Konzentration von mindestens 0,05 μg/kg Körpergewicht/Tag von Ferulasäure liefert, vorzugsweise mindestens 0,06 μg/kg Körpergewicht/Tag von Ferulasäure, besser 0,07 μg/kg Körpergewicht/Tag von Ferulasäure, noch besser 0,08 μg/kg Körpergewicht/Tag von Ferulasäure.

4. Antioxidans-Zusammensetzung nach einem der Ansprüche 1-3, wobei die Antioxidans-Zusammensetzung mindestens 4000 ppm von Resveratrol liefert, vorzugsweise mindestens 4500 ppm Resveratrol, besser mindestens 5000 ppm Resveratrol, besser mindestens 5100 ppm Resveratrol, besser mindestens 5200 ppm Resveratrol, noch besser mindestens 5300 ppm Resveratrol.

5. Antioxidans-Zusammensetzung nach einem der Ansprüche 1 bis 4, die für das Haustier eine Konzentration von mindestens 10 μg/kg Körpergewicht/Tag Resveratrol liefert, vorzugsweise mindestens 15 μg/kg Körpergewicht/Tag Resveratrol, besser 20 μg/kg Körpergewicht/Tag Resveratrol, noch besser 21 μg/kg Körpergewicht/Tag Resveratrol.

6. Antioxidans-Zusammensetzung nach einem der Ansprüche 1 bis 5, wobei das Verhältnis A/B, wobei A Resveratrol ist und B Katechine und/oder Epikatechine sind, mindestens 0,005 beträgt, vorzugsweise mindestens 0,01, besser mindestens 0,02, besser mindestens 0,03, besser mindestens 0,04, besser mindestens 0,05, besser mindestens 0,06, besser mindestens 0,07, besser mindestens 0,08, noch besser mindestens 0,09.

7. Antioxidans-Zusammensetzung nach einem der Ansprüche 1 bis 6, wobei die Antioxidans-Zusammensetzung mindestens 500 ppm von Anthocyaninen enthält, vorzugsweise mindestens 600 ppm von Anthocyaninen, besser mindestens 650 ppm von Anthocyaninen, noch besser mindestens 700 ppm von Anthocyaninen.

8. Antioxidans-Zusammensetzung nach einem der Ansprüche 1 bis 7, die für das Haustier eine Konzentration von mindestens 1,5 μg/kg Körpergewicht/Tag von Anthocyaninen, vorzugsweise mindestens 1,7 μg/kg Körpergewicht/Tag von Anthocyaninen, besser mindestens 2 μg/kg Körpergewicht/Tag von Anthocyaninen, besser mindestens 2,5 μg/kg Körpergewicht/Tag von Anthocyaninen, noch besser mindestens 2,7 μg/kg Körpergewicht/Tag von Anthocyaninen.

9. Antioxidans-Zusammensetzung nach einem der Ansprüche 1 bis 8, wobei die Antioxidans-Zusammensetzung mindestens 50 ppm von Quercetin, vorzugsweise 60 ppm von Quercetin, besser 65 ppm von Quercetin, besser 70 ppm von Quercetin, besser 75 ppm von Quercetin, noch besser 77 ppm von Quercetin enthält.

10. Antioxidans-Zusammensetzung nach einem der Ansprüche 1 bis 9, die für das Haustier eine Konzentration von mindestens 0,1 μg/kg Körpergewicht/Tag von Quercetin liefert, vorzugsweise mindestens 0,2 μg/kg Körpergewicht/Tag von Quercetin , besser mindestens 0,3 μg/kg Körpergewicht/Tag von Quercetin, noch besser 0,37 μg/kg Körpergewicht/Tag von Quercetin.

11. Antioxidans-Zusammensetzung nach einem der Ansprüche 1 bis 10 zur Verwendung in Haustiernahrung, wobei die Haustiernahrung ferner mindestens einen Haustiernahrungsbestandteil enthält, der vorzugsweise ausgewählt wird aus der Gruppe, die besteht aus Proteinen, Peptiden, Aminosäuren, Körnern, Kohlenhydraten, Fetten oder Lipiden, Nährstoffen, Geschmacksverstärker, tierischen Verdauungsmitteln, Fleischmehlen, Gluten, Konservierungsmitteln, Tensiden, Texturierungsmitteln oder Stabilisierungsmitteln, Färbemitteln, anorganischen Phosphatverbindungen, Aromen und/oder Gewürzen.

12. Verfahren zum Herstellen einer Haustiernahrung, umfassend:

i) Bereitstellen einer Antioxidans-Zusammensetzung nach einem der Ansprüche 1 bis 10;
ii) Bereitstellen einer Haustiernahrungszubereitung, die mindestens einen Haustiernahrungsbestandteil enthält;
iii) Zufügen der Antioxidans-Zusammensetzung zur Haustiernahrungszubereitung; und
iv) Gewinnen der Haustiernahrung.

13. Verfahren nach Anspruch 12, wobei die Antioxidans-Zusammensetzung der Haustiernahrungszubereitung in Schritt III) durch Auftragen oder durch Einfügen zugesetzt wird, vorzugsweise durch Einfügen.

14. Zusammenstellung, die in einem oder mehr Behältern in einer einzigen Packung umfasst:

a) einen oder mehrere Haustiernahrungsbestandteile, die vorzugsweise ausgewählt werden aus der Gruppe, die besteht aus Proteinen, Peptiden, Aminosäuren, Körnern, Kohlenhydraten, Fetten oder Lipiden, Nährstoffen, Geschmacksverstärker, tierischen Verdauungsmitteln, Fleischmehlen, Gluten, Konservierungsmitteln, Tensiden, Texturierungsmitteln oder Stabilisierungsmitteln, Färbemitteln, anorganischen Phosphatverbindungen, Aromen und/oder Gewürzen; und
b) eine Antioxidans-Zusammensetzung nach einem der Ansprüche 1 bis 10.

## Revendications

1. Composition antioxydante pour utilisation dans un aliment pour animal de compagnie ou un complément alimentaire pour animal de compagnie comprenant un extrait de *Vitis vinifera* et un extrait de *Vaccinium angustifolium,* dans laquelle ladite composition antioxydante comprend du resvératrol, au moins 1 % de catéchines et/ou épicatéchines, et au moins 5 ppm d'acide férulique,
dans laquelle l'extrait de *Vitis vinifera* est obtenu à partir d'au moins les peaux de *Vitis vinifera,* où ledit extrait de *Vitis vinifera* fournit lesdits resvératrol, catéchines et/ou épicatéchines, et
dans laquelle ledit extrait de *Vaccinium angustifolium* fournit ledit acide férulique.

2. Composition antioxydante selon la revendication 1 qui apporte à l'animal de compagnie une concentration d'au moins 100 $\mu$g/kg p.c./jour de catéchines et/ou épicatéchines, de préférence au moins 150 $\mu$g/kg p.c./jour de catéchines et/ou épicatéchines, plus préférablement 200 $\mu$g/kg p.c./jour de catéchines et/ou épicatéchines, mieux encore 220 $\mu$g/kg p.c./jour de catéchines et/ou épicatéchines.

3. Composition antioxydante selon la revendication 1 ou 2, qui apporte à l'animal de compagnie une concentration d'au moins 0,05 $\mu$g/kg p.c./jour d'acide férulique, de préférence au moins 0,06 $\mu$g/kg p.c./jour d'acide férulique, plus préférablement 0,07 $\mu$g/kg p.c./jour d'acide férulique, mieux encore 0,08 $\mu$g/kg p.c./jour d'acide férulique.

4. Composition antioxydante selon l'une quelconque des revendications 1 à 3, dans laquelle ladite composition antioxydante comprend au moins 4000 ppm de resvératrol, de préférence au moins 4500 ppm de resvératrol, plus préférablement au moins 5000 ppm de resvératrol, plus préférablement au moins 5100 ppm de resvératrol, plus préférablement au moins 5200 ppm de resvératrol, mieux encore au moins 5300 ppm de resvératrol.

5. Composition antioxydante selon l'une quelconque des revendications 1 à 4, qui apporte à l'animal de compagnie une concentration d'au moins 10 $\mu$g/kg p.c./jour de resvératrol, de préférence au moins 15 $\mu$g/kg p.c./jour de resvératrol, plus préférablement 20 $\mu$g/kg p.c./jour de resvératrol, mieux encore 21 $\mu$g/kg p.c./jour de resvératrol.

6. Composition antioxydante selon l'une quelconque des revendications 1 à 5, dans laquelle le rapport A/B, où A représente le resvératrol et B représente les catéchines et/ou épicatéchines, est d'au moins 0,005, de préférence au moins 0,01, plus préférablement au moins 0,02, plus préférablement au moins 0,03, plus préférablement au moins 0,04, plus préférablement au moins 0,05, plus préférablement au moins 0,06, plus préférablement au moins 0,07, plus préférablement au moins 0,08, mieux encore au moins 0,09.

7. Composition antioxydante selon l'une quelconque des revendications 1 à 6, dans laquelle ladite composition antioxydante comprend au moins 500 ppm d'anthocyanines, de préférence au moins 600 ppm d'anthocyanines, plus préférablement au moins 650 ppm d'anthocyanines, mieux encore au moins 700 ppm d'anthocyanines.

8. Composition antioxydante selon l'une quelconque des revendications 1 à 7, qui apporte à l'animal de compagnie une concentration d'au moins 1,5 $\mu$g/kg p.c./jour d'anthocyanines, de préférence au moins 1,7 $\mu$g/kg p.c./jour d'anthocyanines, plus préférablement au moins 2 µg/kg p.c./jour d'anthocyanines, plus préférablement au moins 2,5 µg/kg p.c./jour d'anthocyanines, mieux encore au moins 2,7 µg/kg p.c./jour d'anthocyanines.

9. Composition antioxydante selon l'une quelconque des revendications 1 à 8, dans laquelle ladite composition antioxydante comprend au moins 50 ppm de quercétine, de préférence 60 ppm de quercétine, plus préférablement 65 ppm de quercétine, plus préférablement 70 ppm de quercétine, plus préférablement 75 ppm de quercétine, mieux encore 77 ppm de quercétine.

10. Composition antioxydante selon l'une quelconque des revendications 1 à 9, qui apporte à l'animal de compagnie une concentration d'au moins 0,1 µg/kg p.c./jour de quercétine, de préférence au moins 0,2 µg/kg p.c./jour de quercétine, plus préférablement au moins 0,3 µg/kg p.c./jour de quercétine, mieux encore au moins 0,37 µg/kg p.c./jour de quercétine.

11. Composition antioxydante selon l'une quelconque des revendications 1 à 10 pour utilisation dans un aliment pour animal de compagnie, dans laquelle ledit aliment pour animal de compagnie comprend en outre au moins un ingrédient alimentaire pour animal de compagnie, de préférence choisi dans le groupe constitué par les protéines, les peptides, les acides aminés, les graines, les glucides, les graisses ou les lipides, les nutriments, les facteurs d'appétence, les hydrolysats d'origine animale, les farines de viande, le gluten, les conservateurs, les tensioactifs, les agents de texture ou texturants ou stabilisants, les agents colorants, les composés de phosphate inorganique, les arômes et/ou les assaisonnements.

12. Procédé de préparation d'un aliment pour animal de compagnie, comprenant :

    i) la préparation d'une composition antioxydante selon l'une quelconque des revendications 1 à 10 ;
    ii) l'utilisation d'une préparation alimentaire pour animal de compagnie comprenant au moins un ingrédient alimentaire pour animal de compagnie ;
    iii) l'ajout de ladite composition antioxydante à ladite préparation alimentaire pour animal de compagnie ; et
    iv) l'obtention dudit aliment pour animal de compagnie.

13. Procédé selon la revendication 12, dans lequel ladite composition antioxydante est ajoutée à ladite préparation alimentaire pour animal de compagnie à l'étape iii) par enrobage ou par inclusion, de préférence par inclusion.

14. Kit comprenant, dans un ou plusieurs récipients sous un emballage unique :

    a) un ou plusieurs ingrédients alimentaires pour animal de compagnie, de préférence choisis dans le groupe constitué par les protéines, les peptides, les acides aminés, les graines, les glucides, les graisses ou les lipides, les nutriments, les facteurs d'appétence, les hydrolysats d'origine animale, les farines de viande, le gluten, les conservateurs, les tensioactifs, les agents de texture ou texturants ou stabilisants, les agents colorants, les composés de phosphate inorganique, les arômes et/ou les assaisonnements ; et
    b) une composition antioxydante selon l'une quelconque des revendications 1 à 10.

**Figure 1**

**Figure 2**

| | | Flavonols, µ/L | | Phenolic acids, mg/L | | Flavan-3-ols, mg/L | |
|---|---|---|---|---|---|---|---|
| | | Mean | SEM | Mean | SEM | Mean | SEM |
| **Capsule** | **-1 h** | 1.8 | 0.37 | 9878.4 | 3668.5 | ND | |
| | **0.5h** | 1.4 | 0.27 | 7321.9 | 952.37 | ND | |
| | **1 h** | 2.8 | 0.72 | 6528.8 | 1069.05 | 3.5 | 1.66 |
| | **2 h** | 2.2 | 0.58 | 6501.7 | 1079.27 | 23.5 | 6.6 |
| | **4 h** | 3.2 | 1.18 | 7271.7 | 995.43 | 55.4 | 11.83 |
| | **8 h** | 4.7 | 0.76 | 7816.2 | 1106.1 | 67.6 | 12.23 |
| **Inclusion** | **-1 h** | 0.7 | 0.16 | 4703 | 614.34 | 4.7 | 1.31 |
| | **0.5h** | 1.8 | 0.44 | 5676 | 851.81 | 3 | 1.24 |
| | **1 h** | 2.3 | 0.64 | 6297.7 | 803.47 | 5.2 | 1.57 |
| | **2 h** | 2 | 0.28 | 6208.7 | 650.14 | 23.7 | 3.23 |
| | **4 h** | 4.6 | 0.84 | 7287.3 | 923.32 | 54.2 | 5.5 |
| | **8 h** | 3.1 | 0.68 | 7336.8 | 1183.66 | 42.7 | 9.03 |
| **p-values** | **Mode of administration** | NS | | NS | | NS | |

| | | Stilbenes, mg/L | | Anthocyans, mg/L | | Total phenolic compounds, mg/L | |
|---|---|---|---|---|---|---|---|
| | | Mean | SEM | Mean | SEM | Mean | SEM |
| **Capsule** | **-1 h** | 28.2 | 19.68 | 0.6 | 0.14 | 9909.2 | 3660.8 |
| | **0.5h** | 23.8 | 15.5 | ND | | 7347.5 | 941.62 |
| | **1 h** | 13.3 | 6.57 | 0.5 | 0.12 | 6547.5 | 1064.6 |
| | **2 h** | 10.9 | 4.47 | 0.9 | 0.26 | 6535.3 | 1076.06 |
| | **4 h** | 11.8 | 4.38 | 2.3 | 0.64 | 7344.4 | 996.67 |
| | **8 h** | 13.1 | 4.55 | 3.3 | 0.78 | 7904.9 | 1100.84 |
| **Inclusion** | **-1 h** | 16.1 | 9.4 | 0.3 | 0.08 | 4721.5 | 614.35 |
| | **0.5h** | 18.3 | 12.75 | 0.3 | 0.07 | 5697 | 856.9 |
| | **1 h** | 16 | 7.83 | 0.4 | 0.11 | 6318.9 | 804.16 |
| | **2 h** | 12 | 5.24 | 0.6 | 0.1 | 6247.1 | 651.58 |
| | **4 h** | 10.3 | 2.7 | 1.8 | 0.2 | 7358.2 | 924.2 |
| | **8 h** | 12.7 | 4.94 | 2.5 | 0.37 | 7397.8 | 1186.44 |
| **p-values** | **Mode of administration** | NS | | NS | | NS | |

**Figure 3**

| | | CysC, µg/mL | | Urinary CysC/Creat ratio, µg/g | | Urinary clusterin/Creat ratio, ng/g | | NGAL, ng/mL | | Urinary NGAL/Creat ratio, ng/g | |
|---|---|---|---|---|---|---|---|---|---|---|---|
| | | Mean | SEM | Mean | SEM | Mean | SEM | Mean | SEM | Mean | SEM |
| Week 0 | Control | 1.25 | 0.085 | 18.90 | 17.243 | 79.3 | 20.05 | 17.8 | 4.52 | 11.12 | 4.688 |
| | Mix 1 | 1.25 | 0.152 | 30.38 | 25.390 | 44.5 | 13.22 | 17.0 | 5.02 | 7.97 | 4.487 |
| | Mix 5 | 1.17 | 0.084 | 27.37 | 14.261 | 63.4 | 27.53 | 14.9 | 3.49 | 4.18 | 1.786 |
| | Mix 10 | 1.47 | 0.161 | 12.20 | 4.441 | 83.7 | 22.67 | 18.0 | 3.00 | 5.45 | 1.019 |
| Week 12 | Control | 1.18 | 0.075 | 8.25 | 3.156 | 61.1 | 34.07 | 23.8 | 5.21 | 8.42 | 3.690 |
| | Mix 1 | 1.27 | 0.173 | 9.83 | 4.594 | 67.9 | 33.33 | 15.3 | 4.69 | 5.68 | 1.934 |
| | Mix 5 | 1.22 | 0.079 | 5.12 | 1.696 | 39.5 | 17.16 | 12.9 | 2.80 | 4.00 | 1.551 |
| | Mix 10 | 1.35 | 0.099 | 12.93 | 6.086 | 98.7 | 52.59 | 19.7 | 4.03 | 4.72 | 1.552 |
| Weel 24 | Control | 1.10 | 0.132 | 14.27 | 9.840 | 135.1 | 66.10 | 23.7 | 2.67 | 7.33 | 2.483 |
| | Mix 1 | 1.23 | 0.182 | 4.13 | 1.406 | 94.2 | 69.26 | 19.3 | 4.80 | 4.92 | 1.352 |
| | Mix 5 | 1.18 | 0.079 | 21.42 | 11.941 | 56.2 | 24.72 | 14.3 | 4.04 | 3.52 | 0.297 |
| | Mix 10 | 1.30 | 0.116 | 3.80 | 1.240 | 47.5 | 20.23 | 20.7 | 3.53 | 4.55 | 1.393 |
| p-values | Treatment | NS | | NS | | NS | | NS | | NS | |
| | Day | NS | | NS | | NS | | NS | | NS | |
| | Treatment x Day | NS | | NS | | NS | | NS | | NS | |

**Figure 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2010021533 A
- WO 2007141764 A

### Non-patent literature cited in the description

- **LONDON E D ; OHATA M ; TAKEI H et al.** Regional cerebral metabolic rate for glucose in beagle dogs of different ages. *Neurobiol Aging,* 1983, vol. 4, 121-126
- **MARIA MONAGAS et al.** Commercial dietary ingredients from Vitis vinifera L. leaves and grape skins: antioxidant and chemical characterization. *J. Agric. Food Chem.,* 2006, vol. 54, 2139-327
- **JAYAPRAKASHA et al.** Antioxidant activity of grape seed (vitis vinifera) extracts on peroxidation models in vitro. *Food chemistry,* 2001, vol. 73, 285-290
- **EUBIG P ; BRADY M ; GWALTNEY-BRANT S ; KHAN S ; MAZZAFERRO E ; MORROW C.** *Acute renal failure in dogs after the ingestion of grapes or raisins: a retrospective evaluation of 43 dogs,* 2005, 1992-2002
- **COTMAN CW ; BERCHTOLD NC.** Exercise: a behavioral intervention to enhance brain health and plasticity. *Trends Neurosci.,* 2002, vol. 25 (6), 295-301
- **STUDZINSKI CM ; CHRISTIE L-A ; ARAUJO JA et al.** Visuospatial function in the beagle dog: An early marker of cognitive decline in a model of human cognitive aging and dementia. *Neurobiol Learn Mem,* 2006, vol. 86 (2), 197-204
- **LANDSBERG GM1 ; NICHOL J ; ARAUJO JA.** Cognitive dysfunction syndrome: a disease of canine and feline brain aging. *Vet Clin North Am Small Anim Pract,* July 2012, vol. 42 (4), 749-68
- **TVARIJONAVICIUTE A ; CERON JJ ; HOLDEN SL ; BIOURGE V ; MORRIS PJ ; GERMAN AJ.** Effect of Weight Loss in Obese Dogs on Indicators of Renal Function or Disease. *J. Vet. Intern. Med.,* 2013, vol. 27, 31-38
- **GARCIA-MARTINEZ JD ; TVARIJONAVICIUTE A ; CERON JJ ; CALDEN M ; MARTINEZ-SUBIERLA S.** Urinary Clusterin as a Renal Marker in Dogs. *J. Vet. Diagn. Invest.,* 2012, vol. 24, 301-306